# EUROPÄISCHE PATENTSCHRIFT

(11) **EP 3 433 353 B1**
(45) Veröffentlichungstag und Bekanntmachung des Hinweises auf die Patenterteilung: **28.04.2021**
(21) Anmeldenummer: 17715041.4
(22) Anmeldetag: 16.03.2017
(51) Int. Cl.: C12M 3/00, G01D 11/30

(54) **ANLAGE FÜR BIOTECHNOLOGISCHE ANWENDUNGEN MIT TRÄGER ZUR LAGERUNG VON KOMPONENTEN**
INSTALLATION FOR BIOTECHNOLOGICAL APPLICATIONS, WITH CARRIER FOR THE MOUNTING OF COMPONENTS
INSTALLATION POUR DES UTILISATIONS BIOTECHNOLOGIQUES PRÉSENTANT DES SUPPORTS POUR LOGER DES COMPOSANTS

(30) Priorität: 15.07.2016 DE 102016008655
(43) Veröffentlichungstag der Anmeldung: 30.01.2019
(73) Patentinhaber: Sartorius Stedim Biotech GmbH, 37079 Göttingen (DE)
(72) Erfinder: HEESE, Robin, 36179 Bebra (DE); MIGGE, Andreas, 37081 Göttingen (DE); GREBE, André, 34323 Malsfeld (DE); RODENBERG, Michael, 37574 Einbeck (DE)
(74) Vertreter: Vigand, Philippe
(86) Internationale Anmeldenummer: PCT/EP2017/000342
(87) Internationale Veröffentlichungsnummer: WO 2018/010826

(56) Entgegenhaltungen:
- EP-A1- 2 570 834
- EP-A2- 2 221 518
- EP-A2- 2 274 084
- WO-A1-2007/050971
- WO-A1-2010/109136
- WO-A1-2015/039034
- WO-A1-2015/184189
- DE-A1- 2 633 528
- DE-C1- 3 737 113
- DE-U1-202010 014 864
- GB-A- 2 142 971
- US-A1- 2006 175 472
- US-A1- 2013 101 982
- US-A1- 2013 269 157
- DATABASE WPI Week 201164 Thomson Scientific, London, GB; AN 2011-L65282 XP002771090, & CN 102 155 587 A (HILTI CHINA COMMERCE&TRADE CO LTD) 17. August 2011 (2011-08-17)

## Beschreibung

Die Erfindung betrifft eine Anlage für biotechnologische Anwendungen umfassend mindestens einen Träger zur Lagerung von Komponenten der Anlage. Beispielsweise kann es sich bei der Anlage für biotechnologische Anwendungen, die im Folgenden als Anlage bezeichnet wird, um einen Bioreaktor handeln. Bei der Anlage kann es sich insbesondere um Single-Use-Anlagen für biotechnologische Anwendungen, Crossflow-Anlagen, Bioreaktoren, Biogasanlagen und/oder ähnliche Anlagen handeln. Die in bzw. an der Anlage zu lagernden Komponenten können dabei beispielsweise Sensoren, Filter/Filtersysteme, Leitungen, insbesondere Schläuche und/oder Kabel sein.

Anlagen (z.B. die in WO 2011/134629 oder WO 2009/103450 offenbarten Bioreaktoren) und insbesondere Single-Use-Assemblies umfassen regelmäßig eine hohe Anzahl von Komponenten, welche Leitungen und Sensoren sein können, die an vorbestimmten Positionen geführt bzw. gelagert und/oder fixiert werden und insbesondere dabei nicht in Kontakt mit dem Boden des Raumes, in welchem die Anlage betrieben wird, kommen sollen. Der störungsfreie Betrieb solcher Anlagen erfordert ferner, dass die Komponenten eine spezifische Anordnung einnehmen, bei der die Komponenten insbesondere nicht geknickt oder dauerhaft verformt und/oder beschädigt werden.

GB 2 142 971 A offenbart einen Halter, der es ermöglicht, passende elektrische Kabelverbinder leicht und zuverlässig aneinander zu befestigen. Der Halter umfasst eine starre Klammer und einen selbstschließenden Riemen, wobei die Klammer zwischen den Verbindern und dem von einem Arm ausgehenden Riemen angeordnet ist. Der Riemen ist an einem Ende dauerhaft an dem Arm befestigt, kann um die Verbinder gewickelt und an seinem eigenen Ende mittels Klettverschluss befestigt werden.

DATABASE WPI, Week 201164, Thomson Scientific, London, GB offenbart eine Vorrichtung mit einem konkaven Stützabschnitt, der durch eine Stützplatte gebildet wird. Die Vorrichtung ist ausgelegt zur Aufnahme eines Kabels und eines Abdeckkörpers, welcher an der Stützplatte mittels eines elastischen Schnall-Mechanismus fixiert ist.

EP 2 274 084 A2 offenbart ein Einwegmischgefäß mit einem flexiblen Behälter, einer zentral angeordneten Welle mit einem ersten und einem zweiten Ende mit einem oder mehreren Flügelrädern und einem dem ersten Wellenende zugeordneten Magnetelement, einem ersten Flansch, der zum drehbaren Eingriff mit dem ersten Wellenende ausgebildet ist, und einem zweiten Flansch ausgebildet, um das zweite Wellenende drehbar in Eingriff zu bringen.

WO 2007/050971 A1 offenbart einen Bioreaktor und damit verwandte Verfahren, welche der Bioverarbeitung dienen. Ein Fluid wird dabei unter Verwendung eines internen Fluidrührelements, das von einer externen Bewegungsvorrichtung angetrieben wird, aufgenommen und bewegt. In einer Ausführungsform umfasst der Bioreaktor einen Mischer und einen beweglichen Sprinkler. Der Mischer kann die Form eines Rotationsstabmischers annehmen und kann ferner ein magnetisches Flügelrad umfassen. In einer anderen Ausführungsform umfasst der Bioreaktor einen Rotationsstabmischer und einen integralen Sprinkler. In wieder einer anderen Ausführungsform umfasst der Bioreaktor einen Beutel, der einen Drehmischer mit daran befestigten starren Klingen enthält.

DE 37 37 113 C1 offenbart eine Halteklammer aus hartelastischem Kunststoff zur Befestigung von länglichen Bauteilen mit unterschiedlichen Durchmessern wie Kabel oder Kabelbündel auf Trägerplatten, bestehend aus einer mit einem Befestigungselement versehenen Grundplatte und sich vom Rand der Grundplatte erhebenden, den Halteraum U-förmig umgebenden Seitenwänden, an deren freien Enden den Öffnungsbereich abdeckende Wände mit ineinander verhakbaren Rastkanten angeformt sind.

Bisher werden Leitungen (Schläuche, elektrische Leitungen bzw. Kabel) und Sensoren beim Aufbau oder bei der Wartung von Anlagen mit Hilfe von Kabelbindern oder Klammern (insbesondere Klammern aus Metall und/oder Kunststoff) verlegt und an der Anlage befestigt. Beispielsweise kommen Kabelbinder zum Verlegen, Lagern und Befestigen von Leitungen an solchen Stellen der Anlage zum Einsatz, die es erlauben, den Kabelbinder daran zu binden. Kabelbinder üben aufgrund ihrer schmalen Kontakt- bzw. Auflagefläche einen hohen Druck auf die entsprechende Leitung beim Festziehen des Kabelbinders zur Fixierung der Leitung aus, sodass die Leitung dadurch dauerhaft verformt oder geknickt werden kann. Zum
Entfernen der Verbindung ist zudem ein scharfes Werkzeug, wie eine Schere bzw. eine Klinge nötig, wodurch die Leitung beschädigt werden kann.

Es werden alternativ auch Triclamps, die am Gehäuse oder Profil der Anlage befestigt werden, zur Fixierung von Leitungen verwendet. Zum Schließen der Triclamps muss ein Verbindugsabschnitt festgedreht werden. Triclamps einer vorbestimmten Größe erweisen sich als recht unflexibel bezüglich verschiedener Schlauchformen und -durchmesser. Eine weitere Alternative zur Fixierung von Leitungen bieten Halterungssysteme, in denen Klammern miteinander verschraubt werden, wie beispielsweise Schlauchklemmen. Solche Systeme haben den Nachteil, dass zu deren Bedienung Werkzeug benötigt wird. Oft erweist sich die Bedienung von Werkzeug dadurch als umständlich, da die Schraubenkopfprofile der Schrauben, welche die Klemmen schließen, schwer zugänglich sind. Schlauchklemmen können zudem scharfkantig sein und deren Kanten können die Komponenten bei der Fixierung beschädigen. Weitere Alternativen bilden Federstahlklemmen, Kunststoffklammern oder Fixierringe, Ringe mit Öffnungen, die üblicherweise zur Lagerung von Leitungen verwendet werden. Auch diese Lösungen erweisen sich jedoch in der Praxis als zu umständlich und eine Beschädigung der zu fixierenden Komponente ist nicht ausgeschlossen.

Es ist somit Aufgabe der vorliegenden Erfindung einen effizienten Betrieb bzw. eine effiziente Inbetriebnahme bzw. einen effizienten Aufbau von Anlagen, insbesondere Bioreaktoren und insbesondere Single-Use-Anlagen zu gewährleisten.

Das genannte Problem wird durch die Merkmale der unabhängigen Ansprüche gelöst. Vorteilhafte Ausführungsformen sind Gegenstand der abhängigen Ansprüche.

Gemäß einem Aspekt der vorliegenden Erfindung wird eine Anlage für biotechnologische Anwendungen, insbesondere ein Bioreaktor, bereitgestellt, umfassend zumindest einen Behälter und zumindest einen Träger, wobei der Träger zur Lagerung zumindest einer Komponente der Anlage ausgelegt ist und der Träger dazu ausgelegt ist, direkt oder indirekt an der Anlage angebracht zu werden, und der zumindest eine Träger umfasst zumindest einen Rahmen mit zumindest zwei Armen, zumindest eine Bodenplatte und zumindest ein verformbares Element, wobei das verformbare Element Befestigungsabschnitte umfasst und wobei die Befestigungsabschnitte an den Armen des Rahmens gelagert sind, und wobei das verformbare Element dazu ausgelegt ist, zumindest folgende Zustände einzunehmen: einen Aufnahmezustand, bei dem das verformbare Element zur Aufnahme der Komponente der Anlage bereit steht und einen Arretierzustand, der sich durch das Verlagern der Komponente der Anlage in das verformbare Element ergibt, wodurch das verformbare Element die Komponente der Anlage zumindest teilweise umgibt.

Dementsprechend ist eine Anlage mit verbesserter Befestigungsvorrichtung bzw. verbessertem Halterungssystem zur Führung und/oder Lagerung von Komponenten der Anlage bereitgestellt. Insbesondere können bei Anlagen mit zumindest einer Pumpe die entsprechenden Zu- und/oder Ableitung(en) mittels des zumindest einen Trägers in geeigneter Weise lokalisiert gelagert werden und insbesondere können entstehende Vibrationen durch den zumindest einen Träger gedämpft bzw. im Wesentlichen unterbunden werden. Ferner können insbesondere bei einer Anlage wie einem Bioreaktor und insbesondere einem Single-Use-Assembly auch eine hohe Anzahl von Komponenten, welche Leitung(en) und/oder Sensor(en) sein können, vorteilhaft und einfach an vorbestimmten bzw. vorbestimmbaren Positionen geführt bzw. gelagert und/oder fixiert werden, wodurch insbesondere vermieden werden kann, dass diese in Kontakt mit einem Innenboden der Anlage und/oder dem Boden des Raumes, in welchem die Anlage betrieben wird, kommen. Ferner wird vorteilhaft ein störungsfreier Betrieb solcher Anlagen ermöglicht, da die Komponenten eine spezifische Anordnung einnehmen, bei der die Komponenten insbesondere nicht geknickt oder dauerhaft verformt und/oder beschädigt werden. Zudem kann eine Vielzahl der Komponenten gezielt, geordnet und/oder effizient zu verschiedenen Abschnitten der Anlage, beispielsweise zu Tanks, zu Filtern und/oder zu Pumpen geführt werden. Auch ist es vorteilhaft möglich, insbesondere Sensorsysteme (z.B. Druck- und/oder Durchfluss-Sensoren) an der Anlage anzubringen, im Wesentlichen ohne dass ein physikalischer Druck durch den Träger auf sie ausgeübt wird. Bevorzugt können somit die ein oder mehreren Komponenten einerseits fest und sicher gelagert bzw. gehalten und fixiert werden und andererseits kann eine inkorrekte Beanspruchung (Quetschung und/oder Druck) auf sie zur Vermeidung von Fehlfunktionen und/oder dauerhaften Beschädigungen der Komponenten vermieden werden. Ferner können Vibrationen, beispielsweise ausgelöst durch Pumpen, durch den bzw. die Träger abgefangen werden.

Ferner sind Schlauch-Assemblies bzw. Bausätze umfassend zumindest einen Schlauch und/oder eine andere Komponente der Anlage meist für den einmaligen Gebrauch (d.h. für die Verwendung in einem einzigen Prozess) ausgelegt und werden häufig vom Anwender selbst verlegt. Dementsprechend ermöglicht die Anlage einen schnellen, unkomplizierten und effizienten Ein- und Ausbau, insbesondere unter Vermeidung eines Einsatzes von Werkzeugen, da die (Single-Use-)Komponenten durch Werkzeuge beschädigt werden könnten. Zudem sind Werkzeuge in ihrer Handhabung ineffizient und für den Anwender unkomfortabel (z.B. Drehen, Schrauben oder ähnliches). Daher ist eine werkzeugfreie Lagerung bzw. Anbringung der Komponente(n) an der Anlage mittels des Trägers von Vorteil.

In einer Ausführungsform der Anlage kann die Bodenplatte des Trägers über ein Lager und/oder ein Band und/oder ein Gelenk schwenkbar direkt oder indirekt an der Anlage angebracht sein.

Es besteht ein Vorteil darin, den Träger bzw. die Bodenplatte des Trägers optional über ein Lager und/oder ein Band und/oder ein Gelenk schwenkbar direkt oder indirekt an der Anlage anzubringen, da sich der Träger in die Richtung, in die die Komponente verlegt werden soll, derart ausrichten kann, dass ein Knicken oder Verbiegen der Komponente vorteilhaft vermieden wird. Durch ein Arretieren des Gelenks kann zudem die vorteilhafte Ausrichtung der Komponente (z.B. des Schlauchs bzw. einer Sensoreinrichtung) in die Verlege-Richtung dauerhaft fixiert werden.

In einer Ausführungsform der Anlage kann zumindest einer der Befestigungsabschnitte jeweils über zumindest ein an einem der Befestigungsabschnitte angebrachtes Lagerstück, vorzugsweise ein Gleitlager, um einen Drehpunkt eines an zumindest einem der Arme fixierten Lagergegenstücks, welches sich nicht gegenüber dem jeweiligen Arm verdrehen lässt, gleitend drehbar gelagert sein.

Die optionale gleitend drehbare Lagerung zumindest einer der Befestigungsabschnitte über zumindest ein an einem der Befestigungsabschnitte angebrachtes Lagergegenstück, welches an dem jeweiligen Arm fixiert ist und vorzugsweise ein Gleitlager ist, hat den Vorteil, dass das Lagergegenstück einstückig bzw. integral mit dem Rahmen ausgebildet sein kann. Da der Rahmen vorzugsweise aus einem Metall bzw. Stahl geformt ist, ist das Lagergegenstück bevorzugt ebenfalls aus Metall bzw. Stahl geformt und hat demnach (insbesondere bei einstückiger bzw. integraler Ausbildung mit dem Rahmen) eine hohe Stabilität. Zudem können bei der einstückigen bzw. integralen Ausbildung mit dem Rahmen bei der Herstellung Kosten gespart werden. Insbesondere trifft dies zu, wenn es sich bei dem Lagergegenstück um ein Gelenk oder einen Bolzen handelt. Um eine höhere Stabilität zu erreichen kann zur Herstellung des Gelenks insbesondere ein Metall bzw. Stahl gewählt werden. Es ist ebenfalls denkbar, das Gelenk integral mit dem verformbaren Element auszubilden, wenn dieses aus einem Polymer gefertigt ist, z.B. durch Umgießen.

In einer Ausführungsform der Anlage kann ein Sicherungselement an dem Rahmen und/oder an dem verformbaren Element anordenbar sein, um das Öffnen des verformbaren Elements in dem Arretierzustand zu verhindern.

Durch das optionale Bereitstellen eines zusätzlichen Sicherungselementes wie z.B. eines Bandes und/oder einer Klammer und/oder eines Bügels am Rahmen und/oder am verformbaren Element kann verhindert werden, dass sich das verformbare Element (insbesondere unbeabsichtigt) aus dem Arretierzustand löst und in den Aufnahmezustand übergeht bzw. zu diesem hin verlagert, wodurch die Fixierung bzw. Lagerung der Komponente durch den Träger verschlechtert würde bzw. verloren ginge, was insbesondere durch starke Vibrationen oder starke Durchfluss-Ströme der Flüssigkeiten im Behälter ausgelöst werden könnte.

In einer Ausführungsform der Anlage kann das verformbare Element an seinen Kontaktflächen, die mit der Komponente der Anlage zumindest teilweise in Kontakt stehen, Strukturelemente und/oder Erhöhungen und/oder Vertiefungen umfassen.

Durch zusätzliche optionale Strukturelemente, die z.B. Erhöhungen und/oder Vertiefungen umfassen, können Kontakt- bzw. Druckstellen ausgebildet werden, die im Arretierzustand einen verstärkten Druck bzw. Reibung an ihren Kontaktflächen mit der Komponente erzeugen. Diese Druck- bzw. Reibungsstellen bewirken, dass die Komponente rutschfest gelagert, oder in anderen Worten fester und sicherer von dem verformbaren Element gehalten wird.

In einer Ausführungsform der Anlage kann das verformbare Element dazu ausgelegt sein, Komponenten der Anlage mit unterschiedlichen Formen und/oder Durchmessern bzw. Querschnittsflächen aufzunehmen und/oder zu lagern und/oder zu fixieren.

Ein universeller Gebrauch des Trägers kann dadurch erzielt werden, dass das verformbare Element dazu ausgelegt ist, Komponenten mit unterschiedlichen Formen und/oder Durchmessern aufnehmen zu können. In diesem Fall kann entsprechend ein und der selbe Träger beim Austauschen von unterschiedlichen Komponenten verwendet werden, was den Austausch des Trägers zu einem gewissen Ausmaß überflüssig macht, da der Träger vielseitig verwendet werden kann.

In einer Ausführungsform der Anlage kann zumindest ein von der Anlage umfasstes Element zumindest eine Farbe entsprechend einer Farbcodierung aufweisen.

Ein weiterer Vorteil liegt darin, dass die optionale Farbgebung der einzelnen Elemente der Anlage und insbesondere des Halterungssystems, z.B. des verformbaren Elements, einer Farcodierung unterliegen kann. Die Farbcodierung kann z.B. eine Information zu der Aufnahmefähigkeit von Komponenten eines bestimmten Querschnitts bzw. Durchmessers oder einer bestimmten Form geben. Auch die Flussrichtung von Material durch einen Schlauch kann derart gekennzeichnet werden.

Gemäß einem weiteren Aspekt der vorliegenden Erfindung ist ein Träger bereitgestellt zur Lagerung von mindestens einer Komponente an einer Anlage, umfassend zumindest einen Rahmen mit zumindest zwei Armen, zumindest eine Bodenplatte, und zumindest ein verformbares Element, wobei das verformbare Element Befestigungsabschnitte umfasst und wobei die Befestigungsabschnitte an den Armen des Rahmens gelagert sind und wobei das verformbare Element dazu ausgelegt ist, zumindest folgende Zustände einzunehmen: einen Aufnahmezustand, bei dem das verformbare Element zur Aufnahme der Komponente der Anlage bereit steht, und einen Arretierzustand, der sich durch das Verlagern der Komponente der Anlage in das verformbare Element ergibt, wodurch das verformbare Element die Komponente der Anlage zumindest teilweise umgibt. Daher ist insbesondere ein Träger bereitgestellt, der dazu ausgelegt ist, mindestens eine Komponente einer Anlage zu lagern, und wobei der Träger weiterhin dazu ausgelegt ist, direkt oder indirekt an der Anlage angebracht zu werden.

In einer Ausführungsform des Trägers ist die Bodenplatte über ein Lager und/oder ein Band und/oder ein Gelenk schwenkbar direkt oder indirekt an der Anlage anbringbar.

Gemäß einem weiteren Aspekt der vorliegenden Erfindung ist ein Verfahren bereitgestellt zur Lagerung zumindest einer Komponente an einer Anlage, insbesondere gemäß dem vorangegangenen Aspekt der Erfindung oder eine bevorzugte Ausführungsform hiervon, wobei das Verfahren folgende Schritte aufweist: direktes oder indirektes Anbringen eines Trägers, insbesondere nach dem vorangehenden Aspekt der Erfindung oder eine bevorzugte Ausführungsform hiervon, an der Anlage, welcher zumindest einen Rahmen mit zumindest zwei Armen, zumindest eine Bodenplatte und zumindest ein verformbares Element umfasst, Verlagern der Komponente zu dem verformbaren Element hin, sodass das verformbare Element von einem Aufnahmezustand, in dem das verformbare Element zur Aufnahme der Komponente der Anlage bereit steht, zu einem Arretierzustand hin verlagert wird, wodurch das verformbare Element die Komponente der Anlage zumindest teilweise umgibt und lagert.

Die schnelle und einfache Handhabung einer der Ausführungsformen des Trägers zur wiederholten Verwendung (Verlegen, Lagern, Fixieren, Lösen, Entfernen, Austauschen von Komponenten einer Anlage) per Hand ist vorteilhaft gegenüber der konventionellen Befestigungsmethoden von Komponenten, da der Anwender kein Werkzeug zu deren Bedienung und Verwendung benötigt. Der Anwender kann mit einer einfachen Bewegung eine Komponente "einklicken", wodurch diese dann gelagert bzw. gehalten wird. Erst wenn der Anwender mit etwas Kraft die Komponente aus dem sie zumindest teilweise umgebenden verformbaren Element (senkrecht zur Längsachse der Komponente aus dem Träger heraus-) zieht, löst sich die Komponente aus dem Halter. Das System basiert auf einem einfachen Mechanismus und macht deshalb die Verwendung von Werkzeug überflüssig. Die Komponenten werden deshalb nicht durch die Verwendung von Werkzeug beschädigt. Der Anwender kann zudem sehr effizient und schnell eine Vielzahl von Komponenten verlegen, lagern, fixieren, ausrichten, entfernen und austauschen.

Des Weiteren besteht ein Vorteil in der Verwendung des Trägers darin, dass keine scharfen Kanten oder ähnliche Gefahrenstellen vorliegen, wodurch die zu lagernden und zu fixierenden Komponenten beschädigt werden könnten, oder wodurch der Anwender bei der Bedienung verletzt werden könnte.

Die Komponente der Anlage kann in den Träger eingespannt werden wodurch verhindert wird, dass die Komponenten, z.B. Schläuche locker "durchhängen" und so anfälliger für Störungen bei Vibrationen sind. Dabei werden Vibrationen, Schwingungen und Pulsationen, welche möglicherweise durch Pumpen der Anlage ausgelöst werden, von dem Träger abgefangen und gedämpft. Dies ist insbesondere von Vorteil, wenn der Träger zum Halten von Sensoren verwendet werden soll, da verhindert wird, dass Schwingungen und Vibrationen zwischen unterschiedlichen Komponenten des Sensors übertragen werden. Die Übertragung von Vibrationen auf einen Sensor bzw. einen Sensorkopf könnte das Messsignal beeinflussen, verfälschen, und Störsignale hervorrufen.

Das Halterungssystem und insbesondere der Träger bietet sich alternativ auch vorteilhaft in den folgenden Bereichen an: Laborbedarf, chemische Produktionsanlagen, pharmazeutische Produktionsanlagen, medizinische Diagnosevorrichtungen, Reaktoren im Allgemeinen, Vorrichtungen, die im Allgemeinen auf Prinzipien der Physik basieren und eine hohe Anzahl von Komponenten, wie Leitungen und Kabeln umfassen und Rechenzentren. Insbesondere eignen sich die hierin beschriebenen Ausführungsformen des Trägers und des Halterungssystems zur Verwendung im Zusammenhang mit Single-Use-Anlagen bzw. Single-Use-Systemen.

Ein vielseitiger Einsatz des Trägers für die Halterung und Lagerung von Komponenten, wie von Kabeln, dünnen Filterkapsulen oder kleinen Abgabebehältnissen bietet einen weiteren Vorteil. Zudem können die beschriebenen Ausführungsformen des Trägers überall eingesetzt werden, wo Schläuche, Sensoren, Kabel, (optische und/oder elektrische) Signal-Kabel, Leitungen, Filterkapsulen, kleine Behältnisse oder ähnliche geführt und/oder gelagert werden sollen. Solche Systeme umfassen beispielsweise FlexAct, SartoflowCrossflow-Anlagen, BIOSTAT STR, Pallentank bzw. Fluid-Management.

Weitere Merkmale, Vorteile und Funktionen werden nachfolgend anhand besonderer Ausführungsformen detailliert beschrieben. Obwohl einzelne Ausführungsformen gesondert beschrieben sind, können einzelne Merkmale hiervon zu weiteren Ausführungsformen kombiniert werden
**Figur 1** ist eine schematische teilweise weg-gebrochene Innen- und Außendarstellung einer Anlage gemäß einer ersten Ausführungsform,
**Figur 2a** ist eine perspektivische Rückansicht eines Trägers im Aufnahmezustand,
**Figur 2b** ist eine Explosionsansicht, die im Detail schematisch und beispielhaft die Lagerung eines Befestigungsabschnitts darstellt,
**Figur** 3 ist eine perspektivische Frontalansicht eines Trägers im Aufnahmezustand,
**Figur 4** ist eine perspektivische Frontalansicht eines Trägers im Arretierzustand mit fixiertem Schlauch,
**Figur 5** ist eine schematische beispielhafte Darstellung von Trägern unter Verwendung in Kombination mit Schläuchen und einem Drucksensor,
**Figur 6** ist eine perspektivische Rückansicht eines Trägers im Arretierzustand mit Band als Sicherungselement,
**Figur 7** ist eine perspektivische Rückansicht eines Trägers im Arretierzustand mit Bügel als Sicherungselement, und
**Figur 8** ist eine schematische Außendarstellung einer Anlage gemäß einer weiteren Ausführungsform.

Die Figuren enthalten eingezeichnete Achsen mit Richtungen, die durch Pfeile und Bezugszeichen x, y, z gekennzeichnet sind. Diese Achsen beziehen sich jeweils auf die dargestellten Gegenstände und sind individuell und unabhängig für jede Figur zu verstehen.

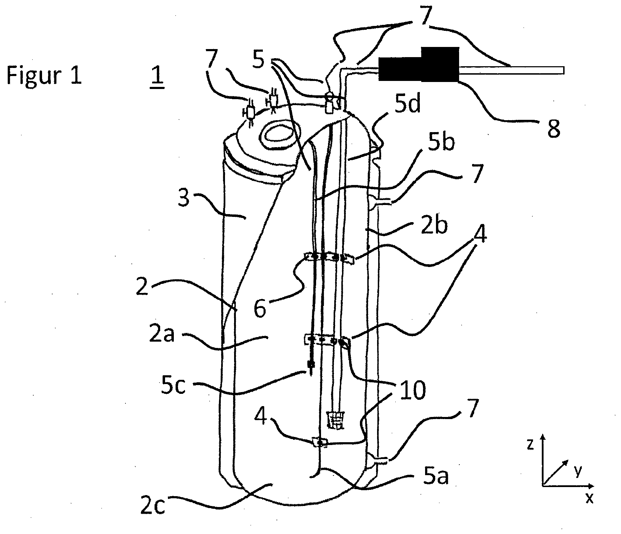

**Figur** 1 zeigt eine (teilweise weg-gebrochene) beispielhafte Außen- und Innenansicht einer Anlage 1. Bei der Anlage 1 kann es sich insbesondere um Single-Use-Anlagen für biotechnologische Anwendungen, Crossflow-Anlagen, Filtrationsanlagen, Bioreaktorbehälter, Biogasanlagen, Mischer bzw. Mischsysteme, Schüttler, Einfrier- und Auftaubehälter, Vorrichtungen für die Behandlung von Fluiden und/oder ähnliche Anlagen handeln. Insbesondere weist eine Anlage für biotechnologische Anwendungen wenigstens eine Pumpe und/oder zumindest eine Transferleitung auf.

Ferner umfassen Anlagen für biotechnologische Anwendungen (z.B. Bioreaktoren) insbesondere Behälter, in denen speziell herangezüchtete Mikroorganismen und/oder Zellen unter möglichst optimalen kontrollierten Bedingungen in einem Nährmedium kultiviert werden, um entweder die Zellen selbst, Teile von ihnen und/oder eines ihrer Stoffwechselprodukte zu gewinnen. Hierzu werden ein oder mehrere Zu- und/oder Ableitungen für die jeweiligen einzelnen Produkte bzw. Stoffe benötigt. Im Speziellen können in den Anlagen feste (Biomasse), flüssige (Nährmedium) und/oder gasförmige (z.B. Luft, Sauerstoff, Kohlendioxid, Stickstoff) Phasen verarbeitet werden.

Um optimale Bedingungen zu gewährleisten, werden üblicherweise ein oder mehrere (verschiedene) Parameter im Innenraum der Anlage mit Hilfe von Sensoren 5c gemessen bzw. überwacht, die in den Innenraum der Anlage hineinragen. Mögliche zu messende Parameter sind beispielsweise der pH-Wert, der O₂-Wert und die Temperatur des in der Anlage enthaltenen Mediums. Sollten Parameter von vordefinierten Optimalwerten abweichen, können mittels geeigneter Maßnahmen die Abweichungen korrigiert werden. Anlagen können zur mehrmaligen Nutzung oder als Einweganlage bzw. -bioreaktor ausgelegt sein. Handelt es sich um eine Einweganlage bzw. eine Einwegbioreaktor, werden diese häufig bereits werkseitig zusammen mit einem oder mehreren Sensor(en) gefertigt, die anschließend als eine sterile Einheit an den Benutzer ausgeliefert wird. Nach Benutzung der Einweganlage bzw. des Einwegbioreaktors, kann der Sensor zusammen mit der Anlage bzw. dem Bioreaktor entsorgt werden. Es handelt sich bei dem Sensor somit um einen Einweg-Sensor.

Die Anlage 1 umfasst einen Behälter 2, insbesondere in Form eines Einwegbeutels, einen Innenraum des Behälters 2a, eine Behälterinnenwand 2b, einen Innenboden 2c des Behälters 2, einen Behälterrahmen 3, ein oder mehrere Pumpen 8 oder Pumpensysteme, ein oder mehrere Zu- und/oder Ausgänge 7, durch die Stoffe dem System ein- oder abgeführt werden können, und/oder zumindest ein Halterungssystem 4.

Das Halterungssystem 4 dient zur zumindest teilweisen Lagerung von einer oder mehreren Komponenten 5 der Anlage 1, insbesondere des Bioreaktors 1, innerhalb und/oder außerhalb des Behälters 2 und/oder am Behälterrahmen 3. Solche Komponenten 5 der Anlage 1 können ein oder mehrere Leitungen, wie z.B. Schläuche 5a, elektrische Kabel 5b und/oder Rohre 5d sein. Die Komponenten 5 können auch Sensoren 5c und/oder Schäfte anderer Komponenten 5 oder Elemente der Anlage 1 umfassen. Das Halterungssystem 4 umfasst mindestens einen oder mehrere Träger 10 bzw. Halter bzw. Halterungen.

Ein Träger 10 ist dazu ausgelegt, eine oder mehrere Komponenten 5 der Anlage 1 zumindest teilweise aufzunehmen, zu lagern und/oder zu fixieren. Der oder die Träger 10 können entweder direkt oder indirekt an der Anlage 1 angebracht bzw. befestigt werden. Beispielsweise kann ein Träger 10 mit der Anlage 1 verklebt (adhäsiv) und/oder verschraubt und/oder magnetisch verbunden werden. Alternativ oder zusätzlich kann ein Träger 10 indirekt an der Anlage 1 angebracht sein. Dazu kann das Halterungssystem 4 optional ein Befestigungssystem 6 umfassen, welches beispielsweise eine Klaue 6a, und/oder ein anderes Verbindungsstück und einer Halterung 6b aufweisen kann. Die Halterung 6b kann z.B. eine Schiene oder eine Halterung 6b mit Klickverbindung aufweisen. Das entsprechende Gegenstück der Halterung kann sich zur Verbindung (insbesondere direkt) am Träger 10, an dem Verbindungsstück des Trägers 10 und/oder an er Klaue 6a befinden. Beispielsweise kann die Halterung 6b einen Zapfen umfassen, der mit einer Öffnung an dem Verbindungsstück wechselwirkt.

Ein Träger 10 wird zur indirekten Anbringung an der Anlage 1 mit einem Verbindungsstück (z.B. der Klaue 6a) verbunden. Der Träger 10 kann dann über das Verbindungsstück, beispielsweise über die Klaue 6a, mit der entsprechenden Halterung 6b, nämlich der Schiene verbunden werden. Die Halterung 6b (hier insbesondere die Schiene) ist dazu an der Anlage 1 angebracht. Die Halterung 6b ist dazu ausgelegt, einen oder mehrere Träger 10 bzw. einen oder mehrere Verbindungsstücke mit den entsprechenden Trägern 10 (bevorzugt verlagerbar bzw. verschiebbar) aufzunehmen. Der Mechanismus, auf dem die Verbindung zwischen Träger bzw. Verbindungsstück und Halterung basiert, umfasst insbesondere: Klickverbindungen, Schienensysteme, Schraubverbindungen, magnetische und/oder adhäsive Verbindungen. Auch die Halterung 6b selbst kann über einen der genannten Mechanismen an der Anlage 1 angebracht sein.

Das Halterungssystem 4 eignet sich insbesondere dazu, Komponenten 5 in einer Single-Use-Anlage zu halten und/oder zu befestigen bzw. zu fixieren. Beispielsweise können mit Hilfe des Halterungssystems 4 eine oder mehrere Komponenten 5 derart an vorbestimmten Positionen verlegt, verlagert und/oder fixiert werden, dass sie dabei insbesondere nicht in Kontakt mit anderen Komponenten 5 und/oder z.B. dem Innenboden des Behälters 2c stehen bzw. kommen. Des Weiteren können die Komponenten 5 auf diese Art effizient, sortiert und/oder platzsparend verlegt werden.

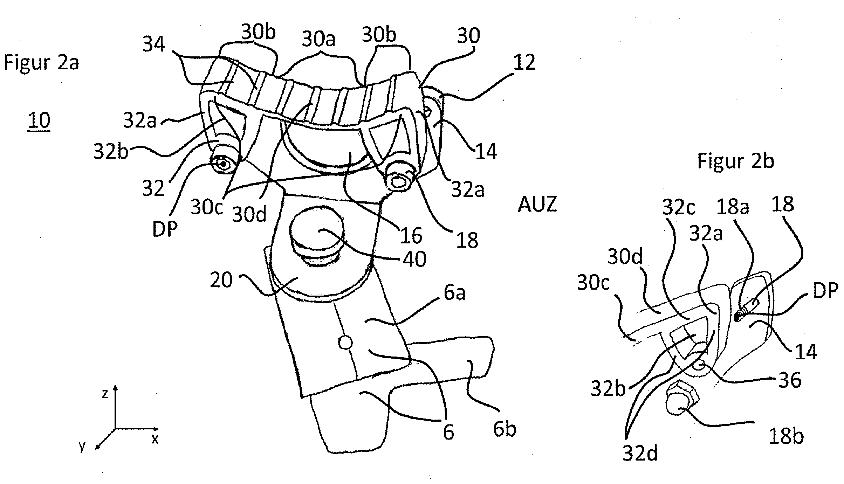

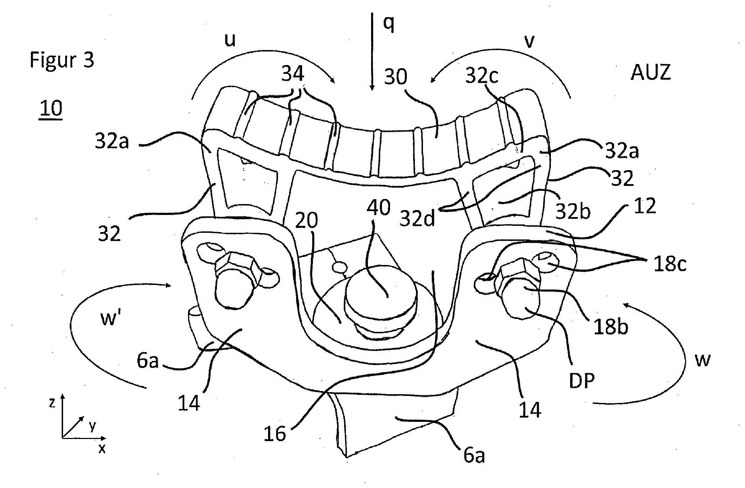

Die **Figuren 2a** **und** **3** stellen jeweils eine perspektivische Rückansicht und eine perspektivische Frontalansicht eines bevorzugten Trägers 10 in einem Aufnahmezustand AUZ dar. Der Träger 10 umfasst einen Rahmen 12. Alternativ kann der Träger 10 auch mehr als einen Rahmen 12 umfassen. Der Rahmen 12 ist im Wesentlichen U- oder alternativ V-förmig ausgebildet und weist zwei Arme 14 auf, die eine Aussparung 16 zumindest teilweise umgeben bzw. definieren. Der Rahmen 12 kann alternativ auch mehr als zwei Arme 14 umfassen, welche mehr als eine Aussparung 16 umgeben.

Der Träger 10 umfasst des Weiteren eine Bodenplatte 20, die sich insbesondere in einem Winkel, der kleiner als 180° und größer als 0° ist (bevorzugt etwa 90° beträgt), von dem Rahmen 12 (insbesondere an dessen distalen Ende entgegengesetzt zu den Armen 14) erstreckt. Der Träger 10 kann alternativ auch ohne Platte bzw. Bodenplatte 20 augebildet sein.

Weiterhin umfasst der Träger 10 ein verformbares Element 30, welches einen verformbaren Abschnitt 30a und zwei starre Abschnitte 30b umfasst. Alternativ kann das verformbare Element 30 auch mehr als einen verformbaren Abschnitt 30a umfassen und/oder nur einen starren Abschnitt 30b oder mehr als zwei starre Abschnitte 30b umfassen.

Das verformbare Element 30 umfasst zudem einen Kontaktabschnitt 30c, vorzugsweise in Form eines Bandes, wobei der Kontaktabschnitt 30c dazu ausgelegt ist, zumindest teilweise über eine Kontaktfläche 30d mit der Komponente 5 der Anlage 1 in Kontakt zu treten. Die Kontaktfläche 30d des verformbaren Elements 30, welche zumindest teilweise mit der Komponente 5 der Anlage 1 in Kontakt tritt, kann ein oder mehrere Strukturelemente 34 enthalten. Solche Strukturelemente 34 können insbesondere Erhöhungen und/oder Vertiefungen, Streben und/oder Noppen, und/oder andere Strukturen aufweisen.

Die Strukturelemente 34 können aber auch lateral, also in der Ebene der Kontaktfläche 30d ausgebildet sein. Mit anderen Worten können die Strukturelemente 34 dem Band bzw. dem Kontaktabschnitt 30c eine seitliche Randstruktur geben. Die Fläche der Kontaktfläche 30d, die (im Arretierzustand ARZ) tatsächlich mit der Komponente 5 in Kontakt steht (bzw. kommen kann), kann sich je nach Ausführung einerseits auf die Flächen der Strukturelemente 34, also die Druckpunkte begrenzen, oder zumindest teilweise mindestens eine zusammenhängende und/oder durchgehende Fläche der Kontaktfläche 30d, die über die Flächen der Strukturelemente 34 hinausgeht, umfassen. In den **Figuren 2a****,** **3****,** **6** und 7 sind die Strukturelemente 34 als Streben ausgebildet, die sich von der Bandfläche bzw. Kontaktfläche 30d erheben und senkreckt zur langen Seite des Bandes erstrecken.

Das verformbare Element 30 in den **Figuren 2a, 2b** **und** **3** ist in dem Aufnahmezustand AUZ dargestellt. Dieser Aufnahmezustand AUZ zeichnet sich insbesondere dadurch aus, dass noch keine Komponente 5 der Anlage 1 von dem Träger 10 aufgenommen ist. Das verformbare Element 30 steht dabei zur Aufnahme einer Komponente 5 der Anlage 1 bereit.

Das verformbare Element 30 umfasst zudem Befestigungsabschnitte 32. Vorzugsweise befinden sich die Befestigungsabschnitte 32 an beiden Enden des Bandes, d.h. an den Enden des Kontaktabschnitts 30c, der zumindest teilweise mit der Komponente 5 der Anlage 1 in Kontakt tritt. Des Weiteren erstrecken sich die Befestigungsabschnitte 32 vorzugsweise in einem Winkel, der größer als 0° und kleiner ist als 180° ist (bevorzugt etwa 90° beträgt), von dem Kontaktabschnitt 30c des verformbaren Elements 30.

Die Befestigungsabschnitte 32 sind jeweils an den Armen 14 des Rahmens 12 vorzugsweise schwenkbar gelagert. **Figur 2b** ist eine perspektivische Detailabbildung eines Befestigungsabschnitts 32, wie er in **Figur 2a** dargestellt ist.

**Figur 2b** stellt beispielhaft schematisch die Lagerung des Befestigungsabschnittes 32 an einem Rahmen 12 dar. Dabei kann mindestens einer der Befestigungsabschnitte 32 um jeweils eine Drehachse bzw. einen Drehpunkt DP dreh- bzw. verlagerbar an jeweils einem der Arme 14 des Rahmens 12 gelagert sein. Vorzugsweise trifft dies für beide Befestigungsabschnitte 32 zu. Dazu umfassen die Befestigungsabschnitte 32 jeweils einen Abschnitt für ein Lagersystem umfassend ein Lagerstück 36, welches mit einem Lagergegenstück 18, und zwar angebracht an dem Rahmen 12, vorzugsweise an einem der Arme 14 des Rahmens 12, kompatibel ist.

Die in **Figur 2b** beispielhaft dargestellte Ausführungsform umfasst ein Lagerstück 36 an dem Befestigungsabschnitt 32, welches in diesem Beispiel eine durchgehende Bohrung bzw. Ausnehmung ist, sowie ein am Rahmen 12 befindliches Lagergegenstück 18, welches in diesem Beispiel insbesondere ein Stift bzw. ein Gelenk bzw. ein Bolzen aufweist. Insbesondere ist das Lagergegenstück 18 aus Metall bzw. Stahl ausgebildet und integral bzw. einstückig mit dem entsprechenden Arm 14 ausgebildet (z.B. ist das Lagerstück 18 mit dem Arm 14 verschweißt).

Das Lagerstück 36 kann aber auch eine (insbesondere verstärkte) Hülse und/oder einen zumindest teilweise ausgesparten Abschnitt bzw. einen Hohlraum bzw. eine Mulde im Befestigungsabschnitt 32 umfassen, der das Lagergegenstück 18 zumindest teilweise umgibt. Das Lagerstück 36 kann zudem auch ein Kugellager, ein Gleitlager und/oder ein ähnliches Lager bzw. einen Gelenk-Aufnahmeabschnitt umfassen. Der Stift bzw. das Gelenk ist vorzugsweise (in der Ausführungsform der **Figur 2b****)** an dem Rahmen 12 fixiert gelagert und bildet somit ein fixiertes nicht drehbares Gelenk, welches sich gegenüber dem Arm 14 nicht drehen kann. Der Stift 18 kann ein Gewinde 18a umfassen und/oder kann zumindest teilweise durch die Durchbohrung bzw. das Lagerstück 36 bzw. den Gelenk-Aufnahmeabschnitt geführt (bzw. darin aufgenommen) werden und insbesondere mittels eines Schließelements 18b, welches vorliegend insbesondere als eine Mutter mit Gewindegegenstück dargestellt ist, verschlossen werden.

Gemäß den **Figuren 2a** **und** **3** umfasst das Gelenk 18 insbesondere eine Schraube, die am Rahmen 12 fixiert ist (z.B. durch Schweißen) und durch eine Mutter 18b an dem Gewinde 18a abgeschlossen wird. Bei Bedarf kann die Position des Lagergegenstücks 18 durch Verschieben auf bzw. entlang eine(r) (insbesondere andere(n)) Verlagerungsaussparung 18c verändert werden. Insbesondere ist die Verlagerungsaussparung 18c als längliches Loch bzw. Langloch bzw. -bohrung ausgebildet. Dies erlaubt, die Kontaktfläche 30d und den Druck auf die Komponente 5 zu variieren. Nach der Aufnahme des verformbaren Elements 30 durch den Stift 18 und nach dem Verschließen durch die Mutter 18b, kann sich der Befestigungsabschnitt 32 insbesondere um die durch den Stift definierte Drehachse bzw. den Drehpunkt DP gegenüber dem Lagergegenstück 18 und dem Rahmen 12 drehen.

Somit kann zumindest einer der Befestigungsabschnitte 32, vorzugsweise aber beide Befestigungsabschnitte 32 des verformbaren Elements 30 jeweils über zumindest ein fixiertes Lagergegenstück 18, das jeweils an zumindest einem der Arme 14 am Rahmen 12 angebracht bzw. fixiert ist, um jeweils einen Drehpunkt DP bzw. Drahachse gleitend drehbar gelagert sein.

Alternativ kann das Lagerstück 36 aber auch entweder einen Stift bzw. Gelenk umfassen und/oder mit einem Stift derart verbunden sein, dass eine Drehung des Befestigungsabschnitts 32 gegen den Stift bzw. das Gelenk nicht möglich ist. Das Lagergegenstück 18 umfasst in diesem alternativen Fall dann einen Aufnahmeabschnitt bzw. einen Gelenkaufnahmeabschnitt, der den Stift bzw. das Lagerstück 36 (zumindest teilweise) dreh- und/oder schwenkbar lagern kann. Der Aufnahmeabschnitt des Lagergegenstücks 18 kann eine Aussparung bzw. eine Durchgangsborhung im Rahmen 12 und/oder ein Kugel- bzw. Gleitlager oder ein ähnliches Lager umfassen. In diesem Fall findet eine Dreh- bzw. Schwenkbewegung des Befestigungsabschnitts 32 mit dem fixierten Stift gegen das Lagergegenstück 18 um einen Dreh- bzw. Schwenkpunkt DP und/oder eine Drehachse statt. Anders gesagt, kann sich das an einem der Arme 14 fixierte Gelenk 36 mit dem Befestigungsabschnitt 32 um den Drehpunkt DP mit drehen. Die dreh-/schwenkbare Lagerung wird durch das Lagergegenstück 18 am Rahmen 12 gewährleistet. Lagerstück 36 und Lagergegenstück 18 können - sofern sie nicht lediglich Aussparungen sind - einen Kunststoff, z.B. ein Polymer und/oder ein Metall oder eine Kombination bzw. eine Mischung daraus umfassen. Beispielsweise kann bei einem Kugellager die Kugel metallisch sein und die Lagerung bzw. das Lager der Kugeln kann aus einem Polymer sein.

Dabei kann zumindest einer der Befestigungsabschnitte 32 zumindest einen Schenkel 32a aufweisen, vorzugsweise umfassen beide Befestigungsabschnitte 32 jeweils zumindest einen Schenkel 32a. Der Schenkel 32a hat außerdem den Abschnitt, der dreh- bzw. verlagerbar an jeweils einem der Arme 14 des Rahmens 12 gelagert bzw. fixiert ist und umfasst demnach eine Drehachse bezüglich des Drehpunktes DP oder der Drehachse und verfügt bei Ausübung einer Drehung über ein Drehmoment. Insbesondere weist der Befestigungsabschnitt 32 zwei Streben bzw. Leisten 32d auf, die in einem Bereich voneinander beabstandet sind und eine Ausnehmung 32b dazwischen ausbilden und sich in einem sich anderen Bereich in Nähe des Gelenks 36 vereinen. Somit bilden die zwei Streben bzw. Leisten 32d mit einem diesen überbrückenden Bereich 32c den starren Abschnitt 30b des verformbaren Elements 30, welcher somit insbesondere eine im Wesentlichen dreieckige Gestalt aufweist.

Es ist ebenfalls möglich, dass der Träger 10 auch dazu ausgelegt ist, mehr als eine Komponente 5 aufzunehmen und zu lagern. Dazu kann der Rahmen 12 insbesondere im Wesentlichen W- und/oder Z-förmig ausgebildet sein und/oder mehr als zwei Arme 14, und entsprechend mehr als eine Aussparung 16 aufweisen. Ferner weist der Träger 10 mehr als ein verformbares Element 30 und/oder ein verformbares Element 30 mit mehr als zwei Befestigungsabschnitten 32 auf. Die benachbarten Enden zweier verformbarer Elemente 30 können dann an einem gemeinsamen Arm 14 gelagert sein.

Alternativ kann sich einer oder beide der Befestigungsabschnitte 32 jedoch auch an dem jeweiligen Ende des Kontaktabschnitts 30c, der mit der Komponente 5 der Anlage 1 zumindest teilweise in Kontakt tritt, direkt befinden, ohne, dass er sich in einem Winkel, der größer als 0° und kleiner als 180° ist (insbesondere etwa 90° beträgt), von diesem erstreckt. In diesem Fall kann der Befestigungsabschnitt 32 derart fixiert sein, dass keine drehbare Lagerung an einem solchen Befestigungsabschnitt 32 vorliegt. Die Aufnahme von Komponenten 5 setzt somit insbesondere keinen Drehmechanismus der Befestigungsabschnitte 32 voraus, sondern erfordert lediglich, dass sich das verformbare Element 30 im Arretierzustand ARZ derart verformt, dass eine stabile Fixierung der Komponente 5 gewährleistet werden kann.

Der verformbare Abschnitt 30a des verformbaren Elements 30 weist mindestens ein Material mit verformbaren, insbesondere flexiblen Eigenschaften auf. Das Material des verformbaren Elements 30 kann ein Kunststoff oder eine Mischung aus Kunststoffen, ein Polymer und insbesondere ein thermoelastisches Elastomer (TPE), aber auch Metalle bzw. Legierungen umfassen. Vorzugsweise ist das verformbare Element 30 aus TPE ausgebildet, da dieses Material eine Materialbeständigkeit gegenüber Säuren und Basen sowie einigen organischen Lösungsmitteln aufweist. Zudem ist TPE beständig gegenüber Reinigung und Sterilisierung per Spülmaschine oder Autoklave bzw. (Dampf-) Sterilisator.

Das verformbare Element 30 kann auch teilweise aus einem der genannten Materialien und teilweise aus einem anderen der genannten Materialien ausgebildet sein. Die Verwendung einer Kombination aus unterschiedlichen Materialien ist insbesondere dann vorteilhaft, wenn ein Abschnitt des verformbaren Elements 30, der vorzugsweise der Befestigungsabschnitt 32 ist, eher starre oder unverformbare Eigenschaften haben soll, während ein anderer Abschnitt, der vorzugsweise der verformbare Abschnitt 30a ist, den der Kontaktabschnitt 30c umfasst und zumindest teilweise mit der Komponente 5 der Anlage 1 in Kontakt tritt, eher verformbare Eigenschaften haben soll. Eine verbesserte Steifigkeit bzw. Starrheit der Befestigungsabschnitte 32 bzw. der Schenkel 32a kann auch dadurch erzielt werden, dass diese in einer stabilen Form ausgebildet sind. Eine solche Form kann z.B. wie in **Figur 3** dargestellt zwei voneinander beabstandete Streben 32d umfassen, die zumindest teilweise eine Aussparung 32b des Schenkels 32a bzw. einen Hohlraum umgeben. Alternativ kann dieser Hohlraum auch gefüllt sein, sodass der Befestigungsabschnitt 32 keine Verstrebungen 32d sondern einen massiven Abschnitt (nicht gezeigt) umfasst.

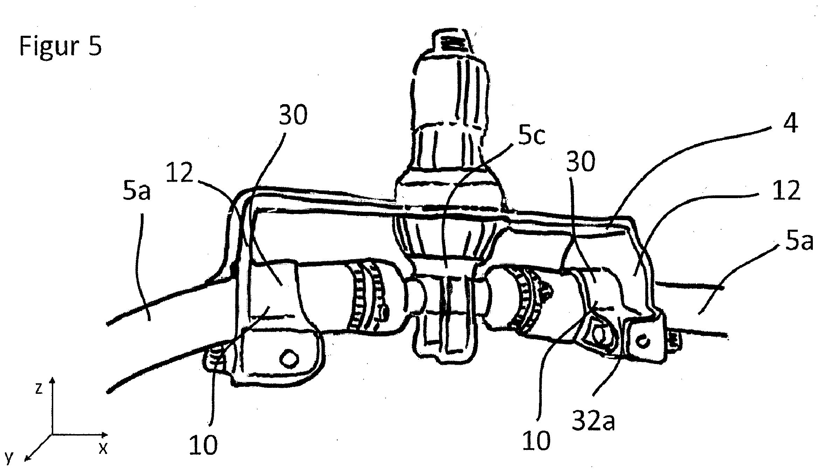

Über die Länge des verformbaren Elements 30 wird der Umfang der (vorzugsweise kreisförmigen) Aufnahme einer Komponente 5 bestimmt, wodurch festlegt wird, welcher Querschnitt bzw. welcher Durchmesser D einer Komponente 5 aufgenommen und gelagert werden kann. Das verformbare Element 30 kann dabei eine Flexibilität bzw. Toleranz bereitstellen, wobei ein verformbares Element 30 einer bestimmten Länge unterschiedlich große Komponenten 5 mit unterschiedlichen Querschnittsflächen lagern kann. Das verformbare Element 30 kann in seiner Abmessung und seiner Form unterschiedlich gestaltet sein, je nachdem welche konkrete Funktion erzielt werden soll. Zum Beispiel ist bei einem einfachen Träger 10 nur ein verformbares Element 30 vorgesehen. Soll dieses Prinzip zum Lagern eines Sensors 5c (z.B. innerhalb einer Schlauchöffnung) verwendet werden, so ist es vorteilhaft, beidseitig von dem Sensor 5c das jeweilige Schlauchende zu halten. Dafür ist es vorteilhaft, wenn der Träger 10 zwei verformbare Elemente 30 hintereinander angeordnet umfasst (vgl. z.B. die nachfolgend näher beschriebene **Figur 5****).** Einer der verformbaren Elemente 30 kann demnach das Schlauchende lagern und das andere verformbare Element 30 könnte den Sensor 5c lagern.

Der Rahmen 12 des Trägers 10 kann aus zumindest einem Kunststoff, Polymer, einem Metall, und/oder einer Legierung, vorzugsweise aus Edelstahl geformt sein und/oder einstückig oder integral ausgebildet sein oder aus mehreren Elementen bestehen. Der Rahmen 12 kann auch aus einer Kombination von Elementen aus unterschiedlichen Metallen, Legierungen und/oder unterschiedlichen Kunststoffen ausgebildet sein. Die Bodenplatte 20 kann ebenfalls einstückig mit dem Rahmen 12 ausgebildet oder sie kann als ein separates Element vorliegen, welches mit dem Rahmen 12 verbunden ist oder verbunden werden kann. Vorzugsweise ist der Rahmen 12 aus Edelstahl ausgebildet, wodurch das Material standhaft gegenüber dem Reinigen und Autoklavieren in Spülmaschinen und Autoklaven bzw. (Dampf-) Sterilisatoren ist.

Der Träger 10 kann mit oder ohne Bodenplatte 20 entweder direkt oder mit Hilfe eines Befestigungssystems 6 an der Anlage 1 angebracht werden. Für den Fall, dass der Träger 10 eine Bodenplatte 20 umfasst, kann die Befestigung über die Bodenplatte 20 erfolgen. Bei der direkten Befestigung des Trägers 10 an der Anlage 1 kann der Träger 10 mit und/oder ohne Bodenplatte 20 mit einem Element der Anlage 1 verschraubt sein, über ein adhäsives Mittel, wie beispielsweise ein Klebstoff, und/oder über ein magnetisches System verbunden und/oder fixiert sein.

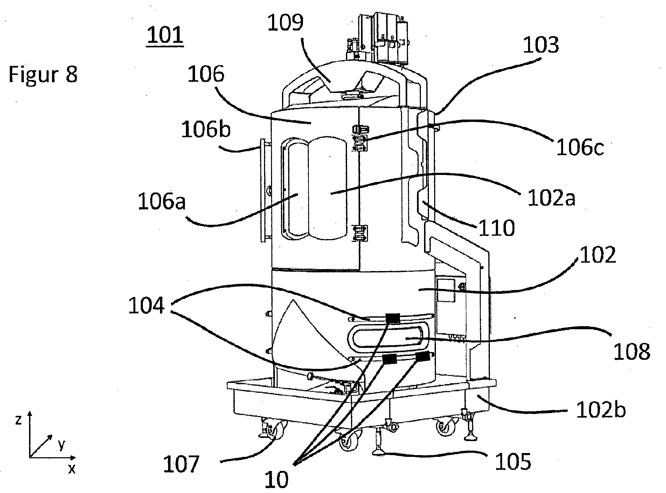

Ein Vorteil der Verwendung einer Bodenplatte 20 besteht in der vereinfachten Handhabung. Beispielsweise können bei der Verschraubung der Bodenplatte 20 an der Anlage 1 Schraubenköpfe in Aussparungen der Bodenplatte 20 versenkt werden. Diese Schraubenköpfe sind entsprechend für den Anwender leicht zugänglich. Soll der Träger 10 über ein Befestigungssystem 6 an der Anlage 1 angebracht werden, so kann beispielsweise der Träger 10 mit und ohne Bodenplatte 20 mit einer Klaue 6a verbunden sein, die an einer Halterung 6b wie z.B. einer Systemschiene 104, wie sie in **Figur 8** dargestellt ist, angebracht und/oder fixiert werden kann. Die Halterung 6b wird dabei direkt oder indirekt an der Anlage 1 angebracht. Das Befestigungssystem 6 kann dabei ein modulares System umfassen, welches dazu ausgelegt ist, eine wechselnde bzw. zuvor nicht festgelegte Anzahl von Trägern 10 aufzunehmen.

Der Träger 10 kann über die Bodenplatte 20 per Schwenkmechanismus, welcher ein Lager, ein Band, und/oder ein Gelenk 40, beispielsweise ein Kugelgelenk umfasst, an dem Befestigungssystem 6 oder der Anlage 1 bzw. einem Element der Anlage 1 schwenk- und/oder drehbar gelagert sein. Wie in **Figur 3** dargestellt, ist der Träger 10 über eine Bodenplatte 20 insbesondere über ein Gelenk 40 dreh- und/oder schwenkbar an einer Klaue 6a der Anlage 1 gelagert. Der Träger 10 kann über diesen Dreh- und/oder Schwenkmechanismus mit oder gegen die Richtung des Uhrzeigersinns (jeweils gekennzeichnet durch die Bezugszeichen w' und w und den dazugehörigen Pfeilen) um die Dreh- bzw. Schwenkachse des Lagers bzw. des Gelenks 40 gegenüber dem Befestigungssystem 6 oder bei direkter Verbindung gegenüber der Anlage 1 gedreht werden. Durch das Drehen und/oder Schwenken ist es vorteilhaft möglich, dass die Komponente 5, die z.B. ein Schlauch 5a sein kann, beim Verlegen in die Verlegerichtung ausgerichtet werden kann, ohne dass die Komponente 5 dabei abknickt. Die erwünschte Ausrichtung der Komponente 5 kann nicht nur verändert, sondern auch durch eine Stell-/Fixierschraube an dem Lager oder Gelenk fixiert werden. Demnach kann eine sehr gezielte Schlauchführung erzielt werden.

Der in **Figur 3** dargestellte Zustand, der dem Aufnahmezustand AUZ entspricht, zeichnet sich dadurch aus, dass sich die Befestigungsabschnitte 32 des verformbaren Elements 30 bei drehbarer Lagerung einander tendenziell abgewandt sind. Dabei ist die Kontaktfläche 30d des Abschnitts, der mit der Komponente 5 der Anlage 1 zumindest teilweise in Kontakt tritt, entgegen der Aussparung 16 (entspricht der z-Richtung der in **Figur 3** dargestellten z-Achse bzw. "nach oben") ausgerichtet. Der Kontaktabschnitt 30c, der über die Kontaktfläche 30d mit der Komponente 5 der Anlage 1 zumindest teilweise in Kontakt tritt, ist allgemein tendenziell in z-Richtung der in **Figur 3** dargestellten z-Achse ("nach oben") ausgerichtet. Die Aussparung 16 des Rahmens 12 ist somit von dem verformbaren Element 30 zumindest teilweise umgeben.

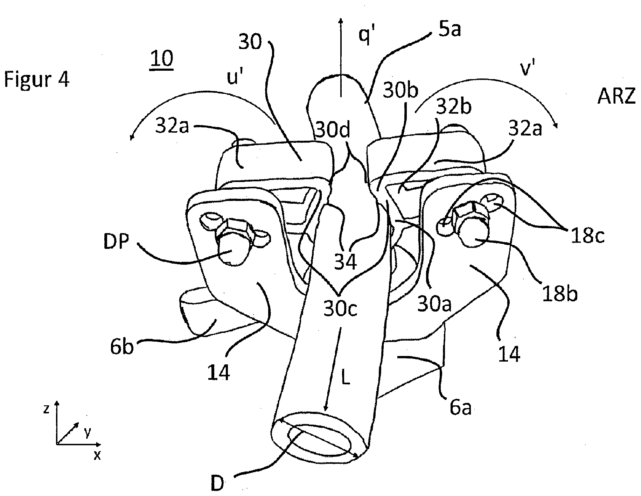

Der Aufnahmezustand AUZ kann in einen weiteren unterschiedlichen Zustand, überführt werden, der dem Arretierzustand ARZ entspricht. Die perspektivische Frontalansicht eines Trägers 10, der sich im Arretierzustand ARZ befindet, ist in **Figur 4** dargestellt. Der Übergang von dem Aufnahmezustand AUZ in den Arretierzustand ARZ kann dadurch erzielt werden, dass eine Komponente 5 der Anlage 1, wie beispielsweise ein Schlauch 5a, (insbesondere im Wesentlichen mittig) auf die Kontaktfläche 30d des Abschnitts, der mit der Komponente 5 zumindest teilweise in Kontakt tritt, in Richtung der Aussparung 16 verlagert bzw. hinein gepresst wird. Die Richtung, in die die Komponente 5 relativ zur Aussparung 16 verlagert wird, ist in **Figur 3** mit einem Pfeil q gekennzeichnet. Der Druck, der über die Komponente 5 auf den Kontaktabschnitt 30c des verformbaren Elements 30 beispielsweise durch einen Anwender ausgeübt wird, löst eine Drehbewegung des Befestigungsabschnitte 32 um die jeweilige Lagerachse bzw. Drehpunkt DP des durch das Lagergegenstück 18 gebildeten Lagers aus. Die Drehung erfolgt für die entsprechenden Befestigungsabschnitte 32 hin zur Aussparung 16. Die Drehrichtungen der Befestigungsabschnitte 32 sind in **Figur 3** mit Pfeilen und den dazugehörigen Bezugszeichen u und v gekennzeichnet. Alternativ können die Befestigungsabschnitte auch von Hand gedreht werden, um den Aufnahmezustand AUZ in den Arretierzustand ARZ zu überführen, beispielsweise zur Demonstration ohne Aufnahme einer Komponente 5. Die **Figur 4** stellt den Zustand dar, in dem die Überführung des Aufnahmezustands AUZ in den Arretierzustand ARZ vollendet wurde. Die Befestigungsabschnitte 32 sind demnach bei vollständiger Überführung von dem Aufnahmezustand AUZ in den Arretierzustand ARZ einander zugewandt.

Der verformbare Abschnitt 30a, der ein Teil des Kontaktabschnitts 30c ist und aus einem Material besteht, das verformbare bzw. flexible Eigenschaften hat, verformt sich als Folge der Drehung der Befestigungsbschnitte 32 und insbesondere des Drucks, der über die Komponente 5 ausgeübt wird. Das verformbare Element 30 im Arretierzustand ARZ hat vorzugsweise eine Krümmung, die tendenziell im Wesentlichen dieselbe Richtung bezüglich des Radius der Krümmung hat wie die Aussparung des Rahmens 12. Das verformbare Element 30 nimmt entsprechend im Arretierzustand ARZ einen gekrümmten Zustand ein. Vorzugsweise bildet das verformbare Element 30 eine im Wesentlichen geschlossene (z.B. kreisförmige) Aufnahme um die Komponente 5 herum aus. Alternativ kann die Krümmung auch eine andere Form und insbesondere im Wesentlichen die Form der jeweiligen Komponente 5 annehmen, welche aufgenommen wurde.

Das verformbare Element 30 kann in einer besonderen Ausführungsform ein Element sein, welches dazu ausgelegt ist, Komponenten 5 mit unterschiedlicher Formen und/oder Querschnittsflächen und/oder Durchmessern D aufzunehmen und zu lagern. Solche Formen der Komponenten 5 umfassen runde, eckige, kantige oder andere Formen. Dazu kann das verformbare Element 30 aus einem Material ausgebildet sein, welches nicht nur über verformbare, sondern insbesondere über dehnbare bzw. flexible Eigenschaften verfügt. Alternativ kann das verformbare Element 30 an dem Kontaktabschnitt 30c, der zumindest teilweise mit der Komponente 5 in Kontakt tritt, aus mehreren Teilabschnitten ausgebildet sein, welche durch einen oder mehrere dehnbare Verbindungsabschnitte mit einander verbunden sind (nicht dargestellt). Dies können beispielsweise Gummiverbindungsstücke bzw. Gummibänder sein. Soll ein Element 5 mit einem größeren Durchmesser D bzw. Querschnitt aufgenommen werden, so kann sich das verformbare Element 30 aufgrund der dehnbaren Eigenschaften dann entsprechend dehnen bzw. verformen und an den aufzunehmenden Gegenstand bzw. die Komponente 5 anpassen.

Das verformbare Element 30 kann alternativ oder zusätzlich auch Sollknickstellen umfassen, welche sich dann im Arretierzustand ARZ knicken bzw. falten. In diesem Fall kann das verformbare Element 30 an dem Kontaktabschnitt 30c auch aus Materialien ausgebildet sein, welche nicht unbedingt über verformbare Eigenschaften verfügen. Die Sollknickstelle bzw. Sollknickstellen und damit verbundenen verformbaren Eigenschaften des verformbaren Elements 30 können dadurch gewährleistet sein, dass das Material an dieser Stelle durch ein verformbares Material, z.B. ein Gummiband, verbunden ist oder das Material in dieser Stelle verjüngt ist oder eine scharnierartige Verbindung von Elementen bereitgestellt wird. Das verformbare Element 30 nimmt entsprechend in diesem alternativen Arretierzustand ARZ einen geknickten bzw. einen gefalteten Zustand ein.

In dem Arretierzustand ARZ ist das verformbare Element 30 derartig verformt, dass ein Abstand zwischen den jeweiligen Befestigungsabschnitte 32 geringer ist als der Abstand der Befestigungsabschnitte 32 in dem Aufnahmezustand AUZ und/oder geringer als ein Außendurchmesser D (bzw. eine Außenabmessung der Komponente 5 quer zur Richtung q) ist, so dass die Komponente 5 durch den Träger 10 zumindest teilweise innerhalb der Aussparung 16 fixiert bzw. (fest) gelagert ist. Hierbei umgibt das verformbare Element 30 die Komponente 5 zumindest teilweise und trägt zur Fixierung bei. Es ist auch denkbar, dass die Befestigungsabschnitte 32 im Arretierzustand ARZ einander kontaktieren. Der Abstand der Befestigungsabschnitte 32 hängt insbesondere von einem Abstand der jeweiligen Drehaxen bzw. -punkte DP, einer radialen Abmessung der Befestigungsabschnitte 32 weg von den jeweiligen Drehaxen bzw. -punkte DP und/oder von einem Ausmaß der Verformung des verformbaren Elements 30 in Richtung q ab.

In dem Arretierzustand ARZ umgibt der Kontaktabschnitt 30c die Komponente 5 über die Kontaktfläche 30d zumindest teilweise kontaktierend. Dabei können die ein oder mehreren Strukturelemente 34 (z.B. Erhöhungen) Druckpunkte auf das verformbare Element 30 bilden, welche zur besseren Fixierung der Komponente 5 beitragen. Je nachdem, wie stark der Druck bei diesen Druckpunkten ist, ist es möglich, dass die Komponente 5 entlang ihrer Längsachse L bzw. entlang der Führungsrichtung verschoben werden kann. Somit ist es beispielsweise bei dem Verlegen von Schläuchen 5a oder Kabeln 5b oder anderen Komponenten 5 möglich, dass die Komponente 5 in ihrer Position (in **Figur 4** entsprechend der x-z-Ebene) fixiert bleibt, wobei sie entlang der Länge L bei Bedarf nachgeführt werden kann. Die Führungsrichtung verläuft im Wesentlichen entlang der Längsachse L der Komponente 5 bzw. im Wesentlichen senkrecht zu der Querschnittsfläche der Komponente 5, die von dem verformbaren Element 30 im Arretierzustand ARZ zumindest teilweise umgeben wird. Diese Querschnittsfläche entspricht in **Figur 4** der Querschnittsfläche des Schlauchs 5a, an dem Abschnitt, der teilweise von dem verformbaren Element 30 umgebenen wird. Im Wesentlichen liegt diese Querschnittsfläche in der Ebene, die in **Figur 4** die x-z-Ebene ist. Die Führungsrichtung entspricht demnach im Wesentlichen der in **Figur 4** dargestellten y-Richtung. Die Komponente 5 der Anlage 1 wird entsprechend durch die Aussparung 16 des Rahmens 12 geführt, wenn sie durch das verformbare Element 30 zumindest teilweise aufgenommen und gelagert ist. Gleichzeitig ist die Komponente 5 derart durch das Halterungssystem 4 fixiert, dass ein Verrutschen, Verschieben oder eine Verlagerung, möglicherweise ausgelöst durch Vibrationen der Anlage, verhindert wird. Die Erfindung ist jedoch nicht beschränkt auf Komponenten 5, die über eine Längsachse L verfügen. Es können auch andere Gegenstände, die nicht verlegt werden, sondern nur gehalten werden, durch das beschriebene Halterungssystem 4 fixiert werden. Solche Gegenstände können z.B. Behältnisse zur Abgabe von Stoffen sein.

Der Arretierzustand ARZ kann bei Bedarf, wenn z.B. die Komponente 5 der Anlage 1 wieder aus der Fixierung in dem Träger 10 herausgelöst werden soll, wieder in den Aufnahmezustand AUZ überführt (bzw. zu diesem hin verlagert) werden, wobei die Komponente 5 von dem verformbaren Element 30 wieder freigegeben wird. Dazu muss eine Kraft entweder auf die Komponente 5 in die der Aussparung 16 entgegengesetzten Richtung (in **Figur 4** durch einen Pfeil q' gekennzeichnet) ausgeübt werden, oder aber es muss eine Kraft bzw. ein Drehmoment, welches direkt an zumindest einem der Befestigungsabschnitte 32 ansetzt, die Drehbewegung der Befestigungsabschnitte 32 in die der Aussparung 16 entgegengesetzten Richtung (in **Figur 4** durch zwei Pfeile u' und v' gekennzeichnet) auslösen.

In **Figur 5** wird die Verwendung von zwei Trägern 10 (vorliegend im Arretierzustand ARZ gezeigt) in Kombination mit einem Drucksensor 5c schematisch beispielhaft dargestellt. Die zu lagernde Komponente 5 ist in **Figur 5** ein Schlauch 5a. Das Halterungssystem 4 umfasst eine Platte 20, die die jeweiligen Rahmen 12 der Träger 10 miteinander verbindet. Die Platte 20 kann dafür einstückig mit den Rahmen 12 der Träger 10 ausgebildet, oder als separates Element mit den Rahmen 12 verbunden sein. Vorzugsweise ist die Platte 20 aus einem Metall geformt. Alternativ können auch mehr als zwei Träger 10 durch eine derartige Platte 20 verbunden sein. Über die Platte 20 kann das Halterungssystem 4 an der Anlage 1 angebracht werden. In **Figur 5** ist auch ein Drucksensor 5c an dem Halterungssystem 4 angebracht. Der Drucksensor misst an einem bestimmten Abschnitt des Schlauchs 5a den Druck, der durch den Fluss bzw. den Strom einer Flüssigkeit durch den Schlauch 5a aufgebaut wird. Alternativ können stattdessen oder zusätzlich auch andere Elemente 5 an dem Halterungssystem gelagert werden.

Vibrationen auf die Schlauchstücke bzw. Abschnittes des Schlauches bzw. der Schläuche 5a, die von den Trägern 10 gelagert werden, können durch die Träger 10 zumindest teilweise abgefangen werden. Diese Ausführungsform ermöglicht es zudem, Abschnitte des Systems bzw. der Anordnung außerhalb der Anlage 1 zu assemblieren und dann nachträglich an der Anlage 1 anzubringen. Die Handhabung beim Verlegen von Komponenten 5 der Anlage 1 wird dadurch wesentlich vereinfacht.

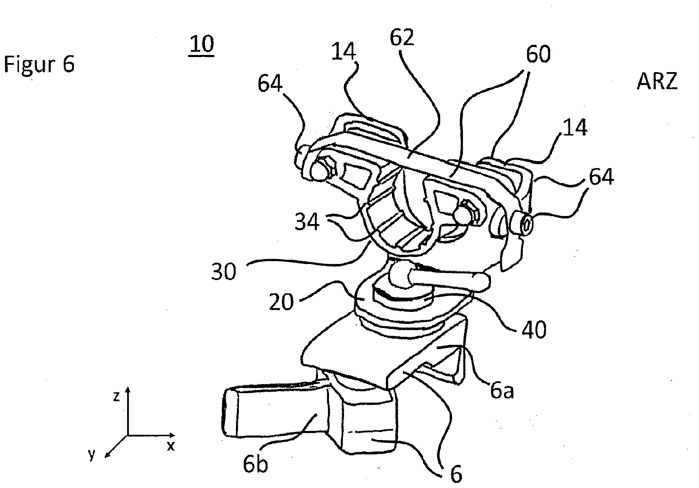

**Figur 6** ist die Rückansicht eines Halterungssystems 4 umfassend einen Träger 10 im Arretierzustand ARZ mit einer optional zu verwendenden zusätzlichen Sicherheitsfunktion und zumindest einem entsprechenden Sicherungssystem 60. Das Sicherungssystem 60 umfasst in der vorliegenden Darstellung ein Band 62 und eine Befestigungsvorrichtung 64. Das dargestellte Halterungssystem 4 liegt zwar im Arretierzustand ARZ vor, hat in dieser **Figur 6** zur demonstrativen Darstellung jedoch keine Komponente 5 aufgenommen und fixiert. Das Sicherungssystem 60 dient zur zusätzlichen Sicherung, die verhindern soll, dass das verformbare Element 30 unbeabsichtigt von dem Arretierzustand ARZ in den Aufnahmezustand AUZ übergeht und somit die Fixierung der Komponente 5 durch das Halterungssystem 4 freigibt. Dies könnte z.B. durch (massive) Vibrationen bzw. einer unbeabsichtigten Bewegung einer Bedienperson ausgelöst werden. Das Sicherungssystem 60 bzw. die Sicherungselemente 60 umfassen ein Band 62 wie in **Figur 6** dargestellt, das vorzugsweise ein Gummiband oder ein dehnbares Element ist.

Das Band 62 wird zur Sicherung über den Abschnitt des verformbaren Elements 30 gelegt, der einen Teilabschnitt hat, welcher wie in **Figur 6** dargestellt, einen Teil der Komponente 5 nicht umgibt oder zumindest keinen Druck in diesem Abschnitt auf einen Teil der Komponente 5 ausüben kann. Die beiden Enden des Sicherungselements 60 können mittels einer Befestigungsvorrichtung 64 (z.B. Schrauben) an (insbesondere jeweiligen Vorsprüngen von) den beiden Armen 14 des Rahmens 12 angebracht sein.

Wie in **Figur 6** dargestellt ist, spannt sich das Band 62 dann über die der Aussparung 16 abgewandten Flächen der beiden Schenkel 32a und den Abschnitt, der die Komponente 5 nicht umgibt, sodass ein ungewolltes Drehen der Schenkel 32a verhindert wird durch den Druck und/oder das dadurch entstehende verstärkende Drehmoment, welches sich beim Anbringen des Bandes 62 ergibt. Zudem umgibt bzw. schließt das Band 62 den Abschnitt der Komponente 5 (insbesondere mechanisch), der entweder nicht von dem verformbaren Element 30 umgeben wird oder der zumindest keinen Druck auf die Komponente 5 in diesem Abschnitt ausübt.

Die Befestigungsvorrichtung 64 kann einstückig mit dem Rahmen 12 ausgebildet oder ein zusätzliches separates Sicherungselement 60 sein, wobei das Sicherungselement 60 an dem Rahmen 12 angebracht, beispielsweise angeklebt oder angeschraubt sein kann. Das Band 62 kann zumindest an einem seiner Enden reversibel durch eine Klemme oder eine Schraube mit Druckfläche mit der Befestigungsvorrichtung 64 verbunden sein. Für den Fall, dass nur ein Ende des Bandes 62 reversibel mit der Befestigungsvorrichtung 64 verbunden ist, kann das andere Ende fest bzw. permanent bzw. irreversibel mit der Befestigungsvorrichtung 64 z.B. adhäsiv verbunden sein. Alternativ oder zusätzlich kann das Sicherungssystem 60 eine (nicht dargestellte) Klammer aufweisen, die die Befestigungsabschnitte 32 überspannt und in dem Arretierzustand ARZ fixiert.

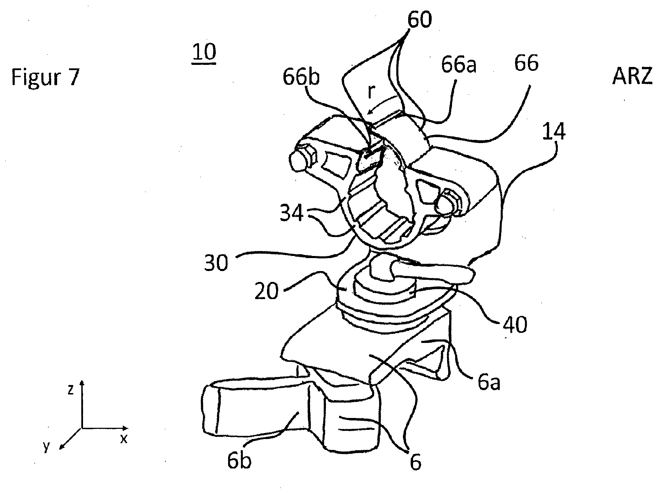

**Figur 7** ist die Rückansicht eines weiteren Halterungssystems 4 umfassend einen Träger 10 im Arretierzustand ARZ mit einer optional anzuwendenden alternativen oder zusätzlichen Sicherheitsfunktion und einem entsprechenden Sicherungssystem 60. Das Sicherungssystem 60 umfasst in der vorliegenden Ausführungsform, anders als in **Figur 6****,** zumindest einen Bügel 66. Der Bügel 66 kann dabei einstückig bzw. integral mit dem verformbaren Element 30 ausgebildet sein oder als separates Element an mindestens eines der Enden des verformbaren Elements 30 angebracht oder anbringbar sein.

Wenn der Bügel 66 nachträglich an beiden Enden des verformbaren Elements 30 durch einen Anwender angebracht werden soll kann der Bügel 66 im Form einer Klemme bzw. einer Klammer ausgebildet sein. Die Klammer 66 kann z.B. zwischen die beiden Schenkel 32a gesteckt bzw. geklemmt werden. Für den Fall, dass der Bügel 66 an mindestens einem Ende des verformbaren Elements 30 nicht permanent angebracht ist, kann der Bügel 66 ein Haken- und/oder Ösensystem, umfassen. Das System besteht z.B. aus einem Haken 66a und dessen Hakengegenstück 66b, welches wiederum ein Haken sein kann. Der Haken 66a und das Hakengegenstück 66b können dabei jeweils an einem Ende des Sicherungsbügels 66 und einem zu verbindenden Ende der Kontaktfläche 30d angebracht sein, und den Bügel 66 in einer verschlossenen Anordnung selektiv fixieren. Der Bügel 66 kann dann an mindestens einem seiner beiden Enden per Haken 66a oder per Öse mit mindestens einem Ende des verformbaren Elements 30, an dem jeweils mindestens ein Gegenstück zu dem Haken 66b oder der Öse angebracht ist, reversibel verbunden werden.

Wie in **Figur 7** dargestellt, erfolgt diese Verbindung durch das Verbiegen bzw. das Umklappen bzw. das Verlagern des Bügels 66 in Richtung der Aussparung 16 des Rahmens 12. Die Biege- bzw. Verlagerungsrichtung des Bügels 66 ist in **Figur 7** durch das Bezugszeichen r und den entsprechenden Pfeil gekennzeichnet. Nach Schließen der Verbindung durch die beiden Haken- und/oder Ösen 66a/66b umgibt bzw. schließt der Bügel 66 den Abschnitt der Komponente 5, der entweder nicht von dem verformbaren Element 30 umgeben wird oder der zumindest keinen Druck auf die Komponente 5 in diesem Abschnitt ausübt.

Die Geschlossene Verbindung der Haken und/oder Ösen 66a/66b verhindert, dass die beiden Befestigungsabschnitte 32 eine ungewollte Drehbewegung ausführen und somit der Arretierzustand ARZ in den Aufnahmezustand AUZ übergeht, wobei die Fixierung der Komponente 5 durch das Halterungssystem 4 versehentlich verloren geht. Sind z.B. zwei Haken 66 a/b in einander verhakt, so kann die Verbindung durch das bloße Ziehen beider durch die Haken 66a/b verbundenen Abschnitte in entgegengesetzte Richtungen nicht aufgehoben werden. Ein solches Ziehen in entgegengesetzte Richtungen tritt z.B. dann auf, wenn ein Drehmoment auf die Befestigungsabschnitte 32 einwirkt und somit ein "Aufdrehen" bzw. Öffnen des Arretierzustandes ARZ auslösen würde, wenn kein zusätzliches Sicherungselement 60 bereitgestellt werden würde. Durch die Hakenverbindung wird dies aus dem genannten Grund allerdings verhindert.

Dadurch, dass der Bügel 66 zum Schließen der Verbindung gebogen werden muss, umfasst der Bügel zur Sicherung 66 Materialien mit verformbaren, insbesondere flexiblen Eigenschaften. Der Bügel 66 kann z.B. aus dem gleichen Material wie das verformbare Element 30 ausgebildet sein. Alternativ kann der Bügel 66 jedoch auch aus allen anderen Materialien, die allgemein für das verformbare Element 30 in Frage kommen, ausgebildet sein.

In **Figur 8** stellt eine weitere Ausführungsform einer Anlage 101 (insbesondere eines Bioreaktors) schematisch dar. Die Anlage 101 umfasst einen Behälter mit einem Innenraum des Behälters 102a und einer Auffangwanne des Behälters 102b, sowie einen Behälterrahmen 103. Die Anlage 101 umfasst zudem eine Tür 106 mit einem Türsichtfenster 106a, einem Türgriff 106b und einem Türscharnier 106c. Fixierfüße 105 sorgen dafür, dass die Anlage 101 an ihrem Bestimmungsort fixiert abgestellt werden kann, wobei diese bei Bedarf in ihrer Höhe verstellt werden können. So kann beispielsweise die Höhe der Fixierfüße 105 derart verstellt werden, dass die Anlage 101 mit ihrem Gewicht darauf lastet, oder die Höhe der Fixierfüße 105 kann derart verringert werden, dass die Anlage 101 auf den Rollen 107 lastet. Die Rollen 107 ermöglichen es dem Verwender, die Anlage 101 an einen anderen Ort zu verschieben bzw. zu rollen. Ein Bodenfenster 108 ermöglicht es dem Verwender, in den Innenraum der Anlage 101 zu blicken, damit dieser den Vorgang darin beurteilen kann, oder feststellen kann, ob sich Materie in der Anlage 101 befindet oder nicht. Zudem ist an der Anlage 101 eine Rührvorrichtung 109 anbringbar, die die Materie bzw. den/die Stoff(e) innerhalb der Anlage 101 verrühren bzw. vermischen kann.

Zumindest eine Kabelführung 110, welche bevorzugt seitlich und/oder oberhalb der Anlage 101 angeordnet und an diesem (direkt oder indirekt) befestigt ist, erlaubt es, Kabel 5b und oder andere Leitungen, wie z.B. Schläuche 5a entlang der Anlage 101 zu ihrem Bestimmungsort zu führen. Zur zusätzlichen Fixierung von ein oder mehreren Komponenten 5 (wie ein Schlauch 5a, ein Kabel 5b, ein Schlauch oder dgl.) können ein oder mehrere oben beschriebene Träger 10 an bzw. bei der Kabelführung 110 angebracht sein.

Des Weiteren umfasst die Anlage 101 insbesondere zumindest eine Systemschiene 104, die als Befestigungssystem 6 für Komponenten 5 der Anlage 101 dienen kann. So kann beispielsweise eine Komponente 5 oder mehrere Komponenten 5 der Anlage 101 direkt oder indirekt über ein oder mehrere Gegenstücke der Systemschiene 104, beispielsweise über Reiter oder Klauen 6a, an und/oder in der Anlage 101 angebracht werden. Die Systemschiene 104 ist dabei insbesondere so ausgebildet, dass sie einen Reiter und/oder eine Klaue 6a entlang einer Leiste führt. Der Reiter und/oder die Klaue 6a umfasst eine Kralle und/oder einen Vorsprung, der entlang einer Aussparung der Schiene geführt bzw. geleitet wird. Alternativ kann die Systemschiene 104 eine Kralle und/oder einen Vorsprung umfassen, die jeweils in eine zu führende Aussparung des Reiters bzw. der Klaue 6a eingreifen. Des Weiteren umfasst das Gegenstück der Systemschiene 104 insbesondere zumindest eine Arretier- bzw. Fixiervorrichtung, die bei Bedienung bzw. Arretieren das Gegenstück an der Systemschiene 104 arretiert bzw. fixiert, sodass ein weiteres Bewegen entlang der Systemschiene 104 verhindert wird.

Die Systemschiene 104 kann dafür ausgelegt sein, eine Vielzahl unterschiedlicher Gegenstücke und/oder unterschiedliche Komponenten 5 an der Anlage 101 anzubringen und/oder zu fixieren. Z.b. ist es möglich über entsprechende Träger 10 eine (nicht gezeigte) Sensorvorrichtung (als eine bevorzugte Komponente 5) derart vor dem Bodenfenster 108 anzubringen, dass die Sensorvorrichtung entsprechende (z.B. optische) Messungen des Inhalts der Anlage 101 vornehmen kann.

Bei Bedarf kann die Position, an der die Komponente 5 fixiert ist, durch Lösen und Verschieben des/der Träger(s) 10 entlang der Systemschiene 104 verändert werden. Die Systemschiene 104 kann an der Anlage 101 adhäsiv und/oder verschraubt und/oder über einen anderen Fixier-Mechanismus, beispielsweise magnetisch angebracht sein. Die Systemschiene 104 ist vorzugsweise aus einem Metall bzw. Stahl ausgebildet, kann aber ein Polymer oder einen anderen Kunststoff umfassen und/oder aus Elementen verschiedener genannter Werkstoffe ausgebildet sein.

Es können jegliche Elemente aller Ausführungsformen der Anlage 1, 101 jeweils eine vorbestimmte Farbe haben und einem Farbcode unterliegen. Beispielsweise können die verformbaren Elemente 30 durch einen Farbcode andeuten, welche Formen und/oder Durchmesser D der Komponenten 5 sie in der Lage sind aufzunehmen. Auch kann die Farbe andere Merkmale anzeigen, wie beispielsweise eine Flussrichtung durch eine Leitung, die Art der Komponente 5, die aufzunehmen ist, oder die Art von Material (beispielsweise eine Flüssigkeit und/oder ein Gas), die durch eine Leitung transportiert werden soll.

In der vorangehenden Erläuterung wird der Begriff des Lagerns von Komponenten 5 verwendet. Das Lagern umfasst hierbei insbesondere sowohl das Halten bzw. die Fixierung, bei der die Komponente 5 je nach Druck entlang ihrer Längsachse L eine Fixierung erfährt, die unter Umständen, z.B. durch ein Ziehen an der Komponente 5, überwunden werden kann, als auch das lose Führen einer Komponente 5, bei dem die Komponente 5 entlang ihrer Längsachse L nicht fixiert wird, sondern lediglich an dem Bestimmungsort, an dem sich der Träger 10 befindet, geführt wird.

Vorzugsweise wird mit der vorliegenden Erfindung eine Fixierung von Komponenten 5 derart erzielt, dass die Komponente 5, wenn sie von dem Träger 10 gelagert wird, ohne äußere Einwirkung entlang ihrer Längsachse L fixiert bzw. sicher und rutschfest gelagert ist. Je nach Druck bzw. Durchmesser D der Komponente 5 bzw. Größe der Kontaktfläche 30d des verformbaren Elements 30 und Art der Strukturelemente 34, kann die Komponente 5 durch leichtes oder stärkeres Ziehen daran entlang ihrer Längsachse L verschoben bzw. nachgeführt werden.

Der Fachmann versteht es, die dargelegten unterschiedlichen bzw. alternativen Ausführungsformen einzelner Elemente des Halterungssystems 4 in beliebigen Kombinationen auszuführen, sofern sie sich nicht gegenseitig ausschließen. Beispielsweise kann ein Träger 10 zwei alternative Sicherungselemente 60, z.B. ein Band 62 und einen Bügel 66 gleichzeitig umfassen.

### Bezugszeichenliste

- 1: Anlage für biotechnologische Anwendungen
- 2: Behälter
- 2a: Innenraum des Behälters
- 2b: Behälterinnewand
- 2c: Innenboden des Behälters
- 3: Behälterrahmen
- 4: Halterungssystem
- 5: Komponente der Anlage
- 5a: Schlauch
- 5b: Kabel
- 5c: Sensor
- 5d: Rohr
- 6: Befestigungssystem
- 6a: Klaue
- 6b: Halterung
- 7: Zu- und/oder Ausgang
- 8: Pumpe
- 10: Träger
- 12: Rahmen
- 14: Arm
- 16: Aussparung des Rahmens
- 18: Lagergegenstück
- 18a: Gewinde
- 18b: Schließelement
- 18c: Verlagerungsaussparung
- 20: Bodenplatte bzw. Platte
- 30: verformbares Element
- 30a: verformbarer Abschnitt des verformbaren Elements
- 30b: starrer Abschnitt des verformbaren Elements
- 30c: Kontaktabschnitt des verformbaren Elements
- 30d: Kontaktfläche des verformbaren Elements
- 32: Befestigungsabschnitt
- 32a: Schenkel
- 32b: Aussparung des Schenkels
- 32c: Überbrückender Bereich des Befestigungsabschnitts
- 32d: Strebe/Leiste des Befestigungsabschnitts
- 34: Strukturelement
- 36: Lagerstück
- 40: Gelenk
- 50: Rohrschelle
- 52: Handschraube
- 60: Sicherungssystem bzw. Sicherungselement
- 62: Band zur Sicherung
- 64: Befestigungsvorrichtung
- 66: Bügel
- 66a: Haken
- 66b: Hakengegenstück
- DP: Drehpunkt
- D: Durchmesser
- L: Längsachse
- ARZ: Arretierzustand
- AUZ: Aufnahmezustand
- u: Drehrichtung
- v: Drehrichtung
- u': Drehrichtung
- v': Drehrichtung
- w: Drehrichtung
- w': Drehrichtung
- q: Bewegungsrichtung
- q': Bewegungsrichtung
- r: Schließrichtung
- 101: Anlage für biotechnologische Anwendungen
- 102: Behälter
- 102a: Innenraum des Behälters
- 102b: Auffangwanne des Behälters
- 103: Behälterrahmen
- 104: Systemschiene
- 105: Fixierfüße
- 106: Tür
- 106a: Türsichtfenster
- 106b: Türgriff
- 106c: Türscharnier
- 107: Rollen
- 108: Bodenfenster
- 109: Rührvorrichtung
- 110: Kabelführung
- x: Richtung im Raum
- y: Richtung im Raum
- z: Richtung im Raum

## Patentansprüche

1. Anlage für biotechnologische Anwendungen (1, 101) umfassend zumindest einen Behälter (2, 102), und zumindest einen Träger (10), wobei der Träger (10) zur Lagerung zumindest einer Komponente (5) der Anlage (1, 101) ausgelegt ist und der Träger (10) dazu ausgelegt ist, direkt oder indirekt an der Anlage (1, 101) angebracht zu werden, und
der zumindest eine Träger (10) umfasst:
zumindest einen Rahmen (12) mit zumindest zwei Armen (14),
zumindest eine Bodenplatte (20) und
zumindest ein verformbares Element (30), wobei das verformbare Element (30) Befestigungsabschnitte (32) umfasst und wobei die Befestigungsabschnitte (32) an den Armen (14) des Rahmens (12) gelagert sind und wobei das verformbare Element (30) dazu ausgelegt ist, zumindest die folgenden Zustände einzunehmen:
einen Aufnahmezustand (AUZ), bei dem das verformbare Element (30) zur Aufnahme der Komponente (5) der Anlage (1, 101) bereit steht; und
einen Arretierzustand (ARZ), der sich durch das Verlagern der Komponente (5) der Anlage (1, 101) in das verformbare Element (30) ergibt, wodurch das verformbare Element (30) die Komponente (5) der Anlage (1, 101) zumindest teilweise umgibt,
wobei zumindest einer der Befestigungsabschnitte (32) jeweils über zumindest ein an einem der Befestigungsabschnitte (32) angebrachtes Lagerstück (36) um einen Drehpunkt (DP) eines an zumindest einem der Arme (14) fixierten Lagergegenstücks (18), welches sich nicht gegenüber dem jeweiligen Arm (14) verdrehen lässt, gleitend drehbar gelagert ist.

2. Anlage für biotechnologische Anwendungen (1, 101) nach Anspruch 1, wobei die Bodenplatte (20) des Trägers (10) über ein Lager und/oder ein Band und/oder ein Gelenk (40) schwenkbar direkt oder indirekt an der Anlage (1, 101) angebracht ist.

3. Anlage für biotechnologische Anwendungen (1, 101) nach einem der vorangehenden Ansprüche, wobei zumindest einer der Befestigungsabschnitte (32) jeweils über zumindest ein an einem der Befestigungsabschnitte (32) angebrachtes Lagerstück (36), das einem Gleitlager entspricht, um einen Drehpunkt (DP) eines an zumindest einem der Arme (14) fixierten Lagergegenstücks (18), welches sich nicht gegenüber dem jeweiligen Arm (14) verdrehen lässt, gleitend drehbar gelagert ist.

4. Anlage für biotechnologische Anwendungen (1, 101) nach einem der vorangehenden Ansprüche, wobei ein Sicherungselement an dem Rahmen und/oder an dem verformbaren Element anordenbar ist, um das Öffnen des verformbaren Elements (30) in dem Arretierzustand (ARZ) zu verhindern.

5. Anlage für biotechnologische Anwendungen (1, 101) nach einem der vorangehenden Ansprüche, wobei das verformbare Element (30) an seinen Kontaktflächen (30d), die mit der Komponente (5) der Anlage (1, 101) zumindest teilweise in Kontakt stehen, Strukturelemente und/oder Erhöhungen (34) und/oder Vertiefungen umfasst.

6. Anlage für biotechnologische Anwendungen (1, 101) nach einem der vorangehenden Ansprüche, wobei das verformbare Element (30) dazu ausgelegt ist, Komponenten (5) der Anlage (1, 101) mit unterschiedlichen Formen und/oder Durchmessern (D) bzw. Querschnittsflächen aufzunehmen und/oder zu lagern und/oder zu fixieren.

7. Anlage für biotechnologische Anwendungen (1, 101) nach einem der vorangehenden Ansprüche, wobei zumindest ein von der Anlage (1, 101) umfasstes Element zumindest eine Farbe entsprechend einer Farbcodierung aufweist.

8. Träger (10) zur Lagerung von mindestens einer Komponente (5) an
einer Anlage (1, 101), umfassend:
zumindest einen Rahmen (12) mit zumindest zwei Armen (14),
zumindest eine Bodenplatte (20) und
zumindest ein verformbares Element (30), wobei das verformbare Element (30) Befestigungsabschnitte (32) umfasst und wobei die Befestigungsabschnitte (32) an den Armen (14) des Rahmens (12) gelagert sind, und wobei das verformbare Element (30) dazu ausgelegt ist, zumindest folgende Zustände einzunehmen:
einen Aufnahmezustand (AUZ), bei dem das verformbare Element (30) zur Aufnahme der Komponente (5) der Anlage (1, 101) bereit steht; und
einen Arretierzustand (ARZ), der sich durch das Verlagern der Komponente (5) der Anlage (1, 101) in das verformbare Element (30) ergibt, wodurch das verformbare Element (30) die Komponente (5) der Anlage (1, 101) zumindest teilweise umgibt,
wobei zumindest einer der Befestigungsabschnitte (32) jeweils über zumindest ein an einem der Befestigungsabschnitte (32) angebrachtes Lagerstück (36) um einen Drehpunkt (DP) eines an zumindest einem der Arme (14) fixierten Lagergegenstücks (18), welches sich nicht gegenüber dem jeweiligen Arm (14) verdrehen lässt, gleitend drehbar gelagert ist.

9. Träger (10) nach Anspruch 8, wobei die Bodenplatte (20) über ein Lager und/oder ein Band und/oder ein Gelenk (40) schwenkbar direkt oder indirekt an der Anlage (1, 101) anbringbar ist.

10. Verfahren zur Lagerung zumindest einer Komponente (5) an einer Anlage (1, 101), wobei das Verfahren folgende Schritte aufweist:
direktes oder indirektes Anbringen eines Trägers (10) an der Anlage (1, 101), welcher zumindest ein Rahmen (12) mit zumindest zwei Armen (14), zumindest eine Bodenplatte (20) und zumindest ein verformbares Element (30) umfasst,
Verlagern der Komponente (5) zu dem verformbaren Element (30) mit einem drehbar an zumindest einem der Arme (14) gelagerten Befestigungsabschnitt (32) hin, so dass das verformbare Element (30) durch Verdrehen des mindestens einen gleitend drehbar gelagerten Befestigungsabschnitts (32) von einem Aufnahmezustand (AUZ), in dem das verformbare Element (30) zur Aufnahme der Komponente (5) der Anlage (1, 101) bereit steht zu einem Arretierzustand (ARZ) hin verlagert wird, wodurch das verformbare Element (30) die Komponente (5) der Anlage (1, 101) zumindest teilweise umgibt und lagert.

## Claims

1. An installation for biotechnological applications (1, 101) comprising at least one container (2, 102) and at least one carrier (10), wherein the carrier (10) is designed for the mounting of at least one component (5) of the installation (1, 101) and the carrier (10) is designed to be attached directly or indirectly to the installation (1, 101), and
the at least one carrier (10) comprises:
at least one frame (12) with at least two arms (14),
at least one base plate (20) and
at least one deformable element (30), wherein the deformable element (30) comprises fastening portions (32) and wherein the fastening portions (32) are mounted on the arms (14) of the frame (12) and wherein the deformable element (30) is designed to assume at least the following states:
a receiving state (AUZ), where the deformable element (30) is ready to receive the component (5) of the installation (1, 101); and
a locked state (ARZ), which is produced by the displacement of the component (5) of the installation (1, 101) into the deformable element (30), whereby the deformable element (30) surrounds at least partially the component (5) of the installation (1, 101),
wherein at least one of the fastening portions (32) is mounted in a slidingly rotatable manner in each case by means at least one bearing part (36) attached to one of the fastening portions (32) about a pivot point (DP) of a bearing counterpart (18) which is fixed to at least one of the arms (14) and which cannot be rotated with respect to the respective arm (14).

2. The installation for biotechnological applications (1, 101) according to claim 1, wherein the base plate (20) of the carrier (10) is attached directly or indirectly to the installation (1, 101) such that it can be pivoted via a bearing and/or a belt and/or a joint (40).

3. The installation for biotechnological applications (1, 101) according to any of the preceding claims, wherein at least one of the fastening portions (32) is mounted in a slidingly rotatable manner in each case by means of at least one bearing part (36) which is attached to one of the fastening portions (32) and corresponds to a sliding bearing about a pivot point (DP) of a bearing counterpart (18) which is fixed to at least one of the arms (14) and which cannot be rotated with respect to the respective arm (14).

4. The installation for biotechnological applications (1, 101) according to any of the preceding claims, wherein a safety element is arrangeable on the frame and/or on the deformable element to prevent the opening of the deformable element (30) in the locked state (ARZ).

5. The installation for biotechnological applications (1, 101) according to any of the preceding claims, wherein the deformable element (30) comprises on its contact surfaces (30d) structural elements and/or elevations (34) and/or indentations which are in contact at least partially with the component (5) of the installation (1, 101).

6. The installation for biotechnological applications (1, 101) according to any of the preceding claims, wherein the deformable element (30) is designed to receive and/or mount and/or fix components (5) of the installation (1, 101) with various forms and/or diameters (D) or cross-sectional surfaces.

7. The installation for biotechnological applications (1, 101) according to any of the preceding claims, wherein at least one element comprised by the installation (1, 101) has at least one color corresponding to a color coding.

8. A carrier (10) for the mounting of at least one component (5) on an installation (1, 101), comprising:
at least one frame (12) with at least two arms (14),
at least one base plate (20) and
at least one deformable element (30), wherein the deformable element (30) comprises fastening portions (32) and wherein the fastening portions (32) are mounted on the arms (14) of the frame (12), and wherein the deformable element (30) is designed to assume at least the following states:
a receiving state (AUZ), where the deformable element (30) is ready to receive the component (5) of the installation (1, 101); and
a locked state (ARZ), which is produced by displacement of the component (5) of the installation (1, 101) into the deformable element (30), whereby the deformable element (30) surrounds at least partially the component (5) of the installation (1, 101),
wherein at least one of the fastening portions (32) is mounted in a slidingly rotatable manner in each case by means at least one bearing part (36) attached to one of the fastening portions (32) about a pivot point (DP) of a bearing counterpart (18) which is fixed to at least one of the arms (14) and which cannot be rotated with respect to the respective arm (14).

9. The carrier (10) according to claim 8, wherein the base plate (20) is attachable directly or indirectly to the installation (1, 101) such that it can be pivoted via a bearing and/or a belt and/or a joint (40).

10. A method for mounting at least one component (5) on an installation (1, 101), wherein the method has the following steps of:
directly or indirectly attaching a carrier (10) to the installation (1, 101), which comprises at least one frame (12) with at least two arms (14), at least one base plate (20) and at least one deformable element (30),
displacing the component (5) toward the deformable element (30) with a fastening portion (32) mounted rotatably on at least one of the arms (14), such that the deformable element (30) is displaced by turning at least one fastening portion (32) mounted in a slidingly rotatable manner from a receiving state (AUZ), in which the deformable element (30) is ready to receive the component (5) of the installation (1, 101), toward a locked state (ARZ), whereby the deformable element (30) surrounds and mounts at least partially the component (5) of the installation (1, 101).

## Revendications

1. Installation pour utilisations biotechnologiques (1, 101) comprenant au moins un récipient (2, 102), et au moins un support (10), dans laquelle le support (10) est conçu pour le logement d'au moins un composant (5) de l'installation (1, 101) et le support (10) est conçu pour être monté directement ou indirectement sur l'installation (1, 101), et
le au moins un support (10) comprend :
au moins un cadre (12) avec au moins deux bras (14),
au moins une plaque de fond (20) et
au moins un élément déformable (30), dans laquelle l'élément déformable (30) comprend des parties de fixation (32) et dans laquelle les parties de fixation (32) sont logées sur les bras (14) du cadre (12) et dans laquelle l'élément déformable (30) est conçu pour occuper au moins les états suivants :
un état de réception (AUZ), pour lequel l'élément déformable (30) est prêt à recevoir le composant (5) de l'installation (1, 101) ; et
un état d'arrêt (ARZ), qui est obtenu par le déplacement du composant (5) de l'installation (1, 101) dans l'élément déformable (30), ce qui a pour effet que l'élément déformable (30) entoure au moins en partie le composant (5) de l'installation (1, 101),
dans laquelle au moins une des parties de fixation (32) est logée de manière à pouvoir tourner en glissant respectivement par l'intermédiaire d'au moins une pièce de palier (36) montée sur une des parties de fixation (32) autour d'un point de rotation (DP) d'une pièce de palier complémentaire (18) fixée sur au moins un des bras (14), laquelle ne peut pas tourner par rapport au bras (14) respectif.

2. Installation pour utilisations biotechnologiques (1, 101) selon la revendication 1, dans laquelle la plaque de fond (20) du support (10) est montée directement ou indirectement sur l'installation (1, 101) de manière pivotante par l'intermédiaire d'un palier et/ou d'une bande et/ou d'une articulation (40).

3. Installation pour utilisations biotechnologiques (1, 101) selon l'une quelconque des revendications précédentes, dans laquelle au moins une des parties de fixation (32) est montée de manière à pouvoir tourner en glissant respectivement par l'intermédiaire d'au moins une pièce de palier (36) montée sur une des parties de fixation (32), qui correspond à un palier lisse, autour d'un point de rotation (DP) d'une pièce de palier complémentaire (18) fixée sur au moins un des bras (14), laquelle ne peut pas tourner par rapport au bras (14) respectif.

4. Installation pour utilisations biotechnologiques (1, 101) selon l'une quelconque des revendications précédentes, dans laquelle un élément de blocage peut être disposé sur le cadre et/ou sur l'élément déformable, afin d'empêcher l'ouverture de l'élément déformable (30) dans l'état d'arrêt (ARZ) .

5. Installation pour utilisations biotechnologiques (1, 101) selon l'une quelconque des revendications précédentes, dans laquelle l'élément déformable (30) comprend sur ses surfaces de contact (30d), qui sont au moins en partie en contact avec le composant (5) de l'installation (1, 101), des éléments structuraux et/ou surélévations (34) et/ou évidements.

6. Installation pour utilisations biotechnologiques (1, 101) selon l'une quelconque des revendications précédentes, dans laquelle l'élément déformable (30) est conçu pour recevoir et/ou pour loger et/ou pour fixer des composants (5) de l'installation (1, 101) avec des formes et/ou diamètres (D) ou surfaces de section transversale différents.

7. Installation pour utilisations biotechnologiques (1, 101) selon l'une quelconque des revendications précédentes, dans laquelle au moins un élément compris par l'installation (1, 101) présente au moins une couleur correspondant à un code couleur.

8. Support (10) pour le logement d'au moins un composant (5) sur une installation (1, 101), comprenant :
au moins un cadre (12) avec au moins deux bras (14),
au moins une plaque de fond (20) et
au moins un élément déformable (30), dans lequel l'élément déformable (30) comprend des parties de fixation (32) et dans lequel les parties de fixation (32) sont logées sur les bras (14) du cadre (12), et dans lequel l'élément déformable (30) est conçu pour occuper au moins les états suivants :
un état de réception (AUZ), pour lequel l'élément déformable (30) est prêt à recevoir le composant (5) de l'installation (1, 101) ; et
un état d'arrêt (ARZ), qui est obtenu par le déplacement du composant (5) de l'installation (1, 101) dans l'élément déformable (30), ce qui a pour effet que l'élément déformable (30) entoure au moins en partie le composant (5) de l'installation (1, 101),
dans lequel au moins une des parties de fixation (32) est logée de manière à pouvoir tourner en glissant respectivement par l'intermédiaire d'au moins une pièce de palier (36) montée sur une des parties de fixation (32) autour d'un point de rotation (DP) d'une pièce de palier complémentaire (18) fixée sur au moins un des bras (14), laquelle ne peut pas tourner par rapport au bras (14) respectif.

9. Support (10) selon la revendication 8, dans lequel la plaque de fond (20) peut être montée directement ou indirectement sur l'installation (1, 101) de manière pivotante par l'intermédiaire d'un palier et/ou d'une bande et/ou d'une articulation (40).

10. Procédé pour le logement d'au moins un composant (5) sur une installation (1, 101), dans lequel le procédé présente les étapes suivantes :
le montage direct ou indirect d'un support (10) sur l'installation (1, 101), lequel comprend au moins un cadre (12) avec au moins deux bras (14), au moins une plaque de fond (20) et au moins un élément déformable (30),
le déplacement du composant (5) en direction de l'élément déformable (30) avec une partie de fixation (32) logée en rotation sur au moins un des bras (14), de sorte que l'élément déformable (30) est déplacé par rotation de l'au moins une partie de fixation (32) logée de manière à pouvoir tourner en glissant d'un état de réception (AUZ), dans lequel l'élément déformable (30) est prêt à recevoir le composant (5) de l'installation (1, 101), en direction d'un état d'arrêt (ARZ), ce qui a pour effet que l'élément déformable (30) entoure et loge au moins en partie le composant (5) de l'installation (1, 101) .
